# EUROPÄISCHE PATENTANMELDUNG

(11) **EP 2 082 755 A1**
(43) Veröffentlichungstag der Anmeldung: **29.07.2009**
(21) Anmeldenummer: 08000745.3
(22) Anmeldetag: 16.01.2008
(51) Int. Cl.: A61L 27/20, A61K 9/00, A61K 8/00, A61P 41/00

(54) **Monolithische in-situ vernetzte Alginatimplantate**

(71) Anmelder: CellMed AG, 63755 Alzenau (DE)
(72) Erfinder: Wallrapp, Christine, 63762 Grossostheim (DE); Glöckner, Herma, 63839 Kleinwallstadt (DE); Reiner, Roland, 64287 Darmstadt (DE); Thürmer, Frank, 63755 Alzenau (DE)
(74) Vertreter: Graf von Stosch, Andreas

(57) **Zusammenfassung**

Die vorliegende Erfindung beschreibt die Verwendung eines Alginats zur Herstellung einer unvernetzten, hochreinen und hochmolekularen Alginatlösung (Sol) als Füllmaterial in der Medizin, insbesondere der (dermatologischen) Chirurgie, oder der Kosmetik zum Zweck der Volumenauffüllung, wobei das Alginat in der unvernetzten, hochreinen und hochmolekularen Alginatlösung (Sol) in einer Konzentration von 0,5-2,5% (w/v) Alginat Feststoffanteil vorliegt und die unvernetzte, hochreine und hochmolekulare Alginatlösung *in vivo* injiziert wird und zur spontanen *in situ* Ca²⁺ Vernetzung ohne exogene Zugabe eines Vernetzers führt. Weiterhin beschreibt die vorliegende Erfindung die Verwendung dieser unvernetzten, hochreinen und hochmolekularen Alginatlösung (Sol) zur Behandlung von Hautfalten, z.B. der Hand, des Gesichts oder des Dekolleté, oder von Volumendefiziten, zur Volumenauffüllung, z.B. bei (HIV-induzierter) Lipoatrophie, der Brüste, und zur Behandlung von ausgewählten Krankheiten, wie z.B. gastroösophagealer Refluxkrankheit, Harninkontinenz oder vesiko-ureteraler Refluxkrankheit, durch Unterspritzung in die entsprechende Sphinktermuskulatur, oder zur Verwendung in der Wiederherstellungschirurgie, insbesondere zu kosmetischen Zwecken.

## Beschreibung

Die vorliegende Erfindung beschreibt die Verwendung eines Alginats zur Herstellung einer unvernetzten, hochreinen und hochmolekularen Alginatlösung (Sol) als Füllmaterial in der Medizin, insbesondere der (dermatologischen) Chirurgie, oder der Kosmetik zum Zweck der Volumenauffüllung, wobei das Alginat in der unvernetzten, hochreinen und hochmolekularen Alginatlösung (Sol) in einer Konzentration von 0,5-2,5% (w/v) Alginat Feststoffanteil vorliegt und die unvernetzte, hochreine und hochmolekulare Alginatlösung *in vivo* injiziert wird und zur spontanen *in situ* Ca²⁺ Vernetzung ohne exogene Zugabe eines Vernetzers führt. Weiterhin beschreibt die vorliegende Erfindung die Verwendung dieser unvernetzten, hochreinen und hochmolekularen Alginatlösung (Sol) zur Behandlung von Hautfalten, z.B. der Hand, des Gesichts oder des Dekolleté, oder von Volumendefiziten, zur Volumenauffüllung, z.B. bei (HIV-induzierter) Lipoatrophie, der Brüste, und zur Behandlung von ausgewählten Krankheiten, wie z.B. gastroösophagealer Refluxkrankheit, Harninkontinenz oder vesiko-ureteraler Refluxkrankheit, durch Unterspritzung in die entsprechende Sphinktermuskulatur, oder zur Verwendung in der Wiederherstellungschirurgie, insbesondere zu kosmetischen Zwecken.

Im Bereich der Medizin bzw. Kosmetik ist insbesondere in den letzten Jahren eine große Nachfrage nach Füllmaterialien entstanden, um Haut- und Muskeleigenschaften aufgrund ästhetischer, krankheits- oder altersbedingter Umstände vorteilhaft durch Volumenvergrößerung zu unterstützen. Solche ästhetischen, krankheits- oder altersbedingten Umstände betreffen u.a. die Bildung von Falten. Falten entstehen bereits im Kindesalter durch Mimik, im späteren Alter typischerweise durch physikalische Schäden wie Sonne, Temperatur, Umwelt und im fortgeschrittenen Alter durch die üblicherweise auftretende Hautalterung. Falten können ebenfalls durch Erkrankungen hervorgerufen werden, die zu einer Verlagerung des Fettgewebes im Körper führen, bspw. einem Fettgewebeschwund mit Rückbildung von Fettgewebe und einer damit einhergehenden Faltenbildung der das Gewebe bedeckenden dermalen Schicht, wie z.B. bei der Lipoatrophie, insbesondere bei HIV-induzierter Lipoatrophie, die zu einem starken Fettgewebeschwund v.a. im Bereich der Extremitäten und der Wangen führt.

Um den anhaltenden Wunsch vieler Patienten nach einem jugendlichen Aussehen nachzukommen, wurden mittlerweile verschiedene Methoden der Faltenbehandlung und Volumenerhöhung der Haut oder des darunter liegenden Gewebes etabliert.

Zum einen kann eine sogenannte chemische Denervierung oder Inaktivierung der betreffenden Muskeln oder Muskelpartien durchgeführt werden. Verwendet werden dabei insbesondere Verbindungen, die die Freisetzung eines Botenstoffes zur Auslösung von Muskelkontraktionen blockieren können, wie z.B. Botulinumtoxin A oder Derivate davon. Bekannte Präparate werden bspw. unter den Namen Botox^{®}, Dysport^{®}, Vistabel^{®} oder Xeomin^{®} vertrieben. Diese bewirken typischerweise im Bereich der behandelten Muskulatur eine Inaktivierung der unterspritzen Muskeln und Muskelpartien, wobei der glättende Effekt, je nach Betätigung der Muskulatur, zwischen wenigen Monaten und einem Jahr anhält. Solche Verfahren tragen daher nur bei regelmäßiger Anwendung zur gewünschten Hautglättung bei.

Andere entwickelte Methoden der Faltenbehandlung basieren auf der Oberflächennivellierung der zu behandelnden Haut durch die Behandlung mit sogenannten "Fillern". Bei Behandlung mit "Fillern" wird die Dermis mit körpereignen oder körperfremden Substanzen unterfüttert und das Gewebe damit stabilisiert und ggf. geglättet. In Europa steht eine große Zahl von körperfremden Fillern zur Verfügung, die überwiegend aus biologischen Substanzen wie Kollagen oder Hyaluronsäure bestehen (z.B.: aus Kollagen: Zyderm^{®}, Zyplast^{®}, Atelocollagen^{®}, Resoplast^{®}. Aus Hyaluronsäure: Hylaform^{®}, Restylane^{®}, Belotero^{®}, Perlane^{®}, Juvederm^{®}, Rofilan Hylan Gel^{®}, Hyal-System^{®}, Viscontur^{®}).

Kollagen ist in diesem Zusammenhang ein natürliches, sowohl in Menschen als auch in Tieren vorkommendes, Protein, welche das (humane) Bindegewebe elastisch hält. Kollagen-Präparate zum Unterspritzen werden typischerweise aus porcinem oder bovinem Kollagen gewonnen. Bei der Verwendung von porcinem und bovinem Kollagen können sich jedoch im Menschen allergische Reaktionen auf diese Eiweißprodukte einstellen, sodass vor der Anwendung notwendigerweise Allergietests durchgeführt werden müssen. Ebenfalls nachteilig an Kollagenpräparaten ist die Tatsache, dass Kollagen von der Injektionsstelle in andere Hautbereiche abwandern kann und dort möglicherweise Rötungen und Schwellungen verursacht (Millikan, 1989, Long term safety and Efficacy with Fibrel in the treatment of cutaneous scars, J Dermatol Surg Oncol, 15:837-842).

Hyaluronsäure, die ebenfalls in körperfremden Fillern enthalten sein kann, ist ein Mucopolysaccharid, das in fast jedem Teil eines lebenden Organismus und insbesondere in der Haut vorkommt. Chemisch wird Hyaluronsäure von geraden Polymerketten mit einem Molekulargewicht im Bereich von mehreren Hunderttausend bis Millionen Dalton gebildet, die sich wiederholende Disaccharideinheiten aus N-Acetylglucosamin und Glucuronsäure enthalten, welche durch glykosidische Bindungen miteinander verknüpft sind. Eine Vergleichsstudie zur Evaluierung der klinischen Verwendbarkeit von Kollagenpräparaten einerseits und Hyaluronsäurepräparaten andererseits hat ergeben, dass das auf Hyaluronsäure basierende Produkt Restylane^{®} deutlich bessere Ergebnisse liefert als das Kollagenpräparat Zyderm^{®} (Narins et al., 2003, A randomized, double-blind, multicenter comparison of the efficacy and tolerability of restylane versus zyplast for the correction of nasolabial folds. Dermatol. Surg., 29: 588-95). Nachteilig an Hyalurosäurepräparaten wirkt sich jedoch aus, dass die Haut für einen sichtbaren Effekt in kurzen Abständen bis zu dreimal behandelt werden muss. Dabei können Schwellungen auftreten, die erst nach 1-2 Tagen abklingen und die Behandlung damit sehr aufwendig gestalten. Sowohl von Hyaluronsäure- als auch Kollagensäurepräparaten sind zudem Komplikationen zwei bis drei Jahre *post injectionem* bekannt - zu einem Zeitpunkt, bei dem die eingespritzten Materialien längst abgebaut waren (Hanke et al., 1991, Abscess formation and local necrosis after treatment with Zyderm or Zyplast Collagen Implant. Journal of American Academy of Dermatology, 25 (No 2, Part 1): 319-26; Moscona et al., 1993, An unusual late reaction to Zyderm I injections: A challenge for treatment. Plastic and reconstructive surgery, 92: 331-4). Abschließend erzeugt der menschliche (und tierische) Körper Enzyme, die Hyaluronsäure abbauen. Eine Behandlung ausschließlich mit Hyaluronsäure oder mit Präparaten, die im wesentlichen oder zu einem großen Bestandteil Hyaluronsäure enthalten, führt daher zu einem schnellen Abklingen des sichtbaren Effekts und erfordert typischerweise eine verhältnismäßig häufige Wiederholung der Behandlung. Heutzutage wird Hyaluronsäure vielfach als venetzte Hyaluronsäure eingesetzt. Dadurch wird zwar der Effekt des Abbaus von natürlicher Hyaluronsäure im Gewebe und das schnelle Abklingen des sichtbaren Effekts der Volumenauffüllung vermindert, jedoch sind die eingesetzten venetzten Hyaluronsäureverbindungen typischerweise chemisch massiv veränderte Hyaluronsäuren, die keine "natürlichen Filler" mehr darstellen.

Als Alternative zur Verwendung von oben genannten körperfremden Fillern wie Kollagen oder Hyaluronsäure zur Faltenunterspritzung wurde ebenfalls lange Zeit flüssiges Silikon eingesetzt. Hierbei haben sich jedoch auch zahlreiche nachteilige Nebenwirkungen eingestellt, wie z.B. die Bildung von Knötchen, periodisch wiederkehrende Zellulitis und die Bildung von Hautgeschwüren. Daher gilt die Behandlung mit Silikon nicht mehr als empfehlenswert (z.B. Edgerton et al., 1976, Indications for and pitfalls of soft tissue augmentation with liquid silicone, Plast. Reconstr. Surg. 58: 157-65).

Als weitere Alternative von körperfremden Fillern wurden jüngst Implantate auf Basis von vernetzten Alginaten zur Faltenunterspritzung in die Haut entwickelt und deren Verwendung zur Behandlung von Falten erfolgreich getestet (siehe WO 2005/105167, Cellmed AG, Deutschland). Dabei konnten v.a. die vorgenannten Probleme verhindert werden, z.B. die Notwendigkeit einer mehrfachen Behandlung zur Erzielung eines sichtbaren Effekts, das Auftreten von Schwellungen, die erst 1-2 Tage nach der Injektion bzw. Implantation abklingen. So beschreibt z.B. die veröffentlichte Internationale Patentanmeldung WO 2005/105167 (Cellmed AG, Deutschland) Implantate auf Basis von vernetzten Alginaten zur Faltenunterspritzung in die Haut, die aufgrund der geringen Immunogenität der Alginate eine wesentlich bessere Verträglichkeit des injizierten Füllmaterials gewährleisten. WO 2005/105167 beschreibt insbesondere die Verwendung von vernetzten Alginaten in Form von implantierbaren Mikrokapseln oder Mikropartikeln oder von Gelen aus mit zwei- oder mehrwertigen Kationen vernetzten Alginaten für die Anwendung als "Filler"-Substanz und in der Behandlung von Hautdefiziten, wie z.B. Falten. Solche implantierbaren Mikrokapseln oder Mikropartikel führen insbesondere nicht zu allergischen Reaktionen oder einer körpereigenen Immunantwort des Patienten. Ungeachtet ihrer Vorteile muss, falls das Alginat erst *in situ* vernetzt werden soll, ein Vernetzer parallel zugeführt werden, was eine Doppelkanüle und damit eine verhältnismäßig aufwendige Vorbereitung der Verabreichung erfordert. Bevorzugt wäre es daher, im Bereich der Faltenunterspritzung solche Stoffe und Materialien bereitzustellen, die keinen solchen Aufwand erfordern und sich ggf. auch in größeren Volumen injizieren lassen.

Solche, wie oben beschriebenen, körpereigenen oder körperfremden Füllmaterialien sind jedoch nicht nur aus kosmetischen Anwendungen bekannt, sondern können auch eingesetzt werden um ausgewählte Krankheiten durch Unterspritzung in die entsprechende Sphinktermuskulatur zu behandeln, wie z.B. die gastroösophageale Refluxkrankheit, Harninkontinenz oder die vesiko-ureterale Refluxkrankheit.

Obwohl die gastroösophageale Refluxkrankheit (Gastroesophageal reflux disease, "GERD") ein normales physiologisches Phänomen darstellt, kann sie zu schweren pathophysiologischen Symptomen führen. Die gastroösophageale Refluxkrankheit beschreibt den Rückfluss von saurer, enzymatischer Flüssigkeit aus dem Magen in den Ösophagus. Sie verursacht Sodbrennen, Aufstoßen und Erbrechen der Magensäure in den Mundraum oder sogar in die Lunge. Die Folgen der gastroösophagealen Refluxkrankheit sind Verätzungen der Speiseröhre und die Bildung von Geschwüren, wobei das normale Epithelgewebe durch pathologisches Gewebe ersetzt wird. Bei gesunden Patienten schließt sich nach der Nahrungsaufnahme der untere ösophageale Sphinktermuskel. Bei Patienten die an der gastroösophagealen Refluxkrankheit leiden, passiert dies nicht. Stattdessen relaxiert typischerweise der Muskel und die Magensäure kann bei Magenkontraktion in die Speiseröhre fließen. Neben dieser Hauptursache für "GERD" sind auch andere Ursachen möglich und bekannt.

Die gastroösophageale Refluxkrankheit (Gastroesophageal reflux disease, "GERD") ist weit verbreitet. So belegen statistische Daten, dass ungefähr 35% der amerikanischen Bevölkerung mindestens einmal im Monat und etwa 5 bis 10% darunter einmal am Tag an Sodbrennen leiden. Medizinisch gesicherte endoskopische Untersuchungen zeigen, dass 2% der amerikanischen Bevölkerung unter "GERD" leiden. Das Risiko, an "GERD" zu erkranken steigt ab dem 40. Lebensjahr (Nebel et al., 1976, Symptomatic gastroesophageal reflux: incidence and precipitating factors, Am. J. Dig. Dis., 21: 953-6). Erste Anzeichen der gastroösophagealen Refluxkrankheit sind üblicherweise endoskopisch sichtbare Rötungen. Ein fortgeschrittener Krankheitsverlauf lässt sich an der Zerstörung des Gewebes, gefolgt von Geschwulstbildung bis hin zum Karzinom (Adenokarzinom des Ösophagus) erkennen. Eine diffuse Geschwulstbildung tritt bei 3,5% der Patienten unter dem 65. Lebensjahr und bei 20-30% der Patienten über dem 65. Lebensjahr auf (Reynolds, 1996, Influence of pathophysiology, severity, and cost on the medical management of gastroesophageal reflux disease. Am J. Health-Syst. Pharm 53:5-12).

Zur Zeit wird "GERD" im allgemeinen mit Protonenpumpeninhibitoren behandelt, mit deren Hilfe bei ausreichender Dosierung der Großteil der Patienten erfolgreich behandelt werden kann. Nachteilig wirkt sich dabei jedoch aus, dass aufgrund der hohen Rezidivhäufigkeit nach Absetzen der säuresuppressiven Therapie bei den meisten Patienten für eine dauerhafte konservative Beseitigung der Symptome eine medikamentöse Dauertherapie erforderlich ist (Bittinger und Messmann, 2003, Neue endoskopische Therapieverfahren bei gastroösophagealer Refluxkrankheit, Z. Gastroenerol, 41: 921-8). Viele Patienten sind zudem nicht bereit, über Jahrzehnte hinweg täglich Medikamente einzunehmen. Hinzu kommt noch die Problematik der nicht unbeträchtlichen Kosten einer solchen medikamentösen Dauertherapie.

Neben der offenen und laparaskopischen Fundoplikation, kommen in jüngster Zeit auch endoskopische Therapieverfahren zum Einsatz mit dem Ziel, die Hauptursache der gastroösophagealen Refluxkrankheit, nämlich den inkompetenten unteren Ösophagussphinkter, therapeutisch anzugehen. Dabei werden zumeist 3 verschiedene Grundprinzipien verfolgt. Zum Einen können Nahttechniken (z.B. endoskopische Gastroplastie, Vollwandoplikation) eingesetzt werden. Weiterhin ist eine Radiofrequenz-Applikation möglich und zum Dritten können Injektions- und Implantationsverfahren (z.B. Injektion von körpereigenen oder körperfremden Füllmaterialien, Biopolymerinjektion, oder Implantationstherapie) angewendet werden.

Solche Injektions- und Implantationsverfahren umfassen u.a. auch die Injektion von körpereigenen oder körperfremden Füllmaterialien. Versuche, die Sphinktermuskulatur durch Unterspritzen von quellbaren Substanzen als natürliche konventionelle Füllmaterialien zu unterstützen, z. B. die Verwendung von bovinem Kollagen oder Teflonpaste, schlugen jedoch leider fehl, weil das Material im Laufe der Zeit vom ursprünglichen Injektionsort abwanderte bzw. resorbiert wurde und eine dauerhafte Behandlung somit nicht ermöglichte.

Eine weitere Alternative solcher Injektions- und Implantationsverfahren (z.B. Biopolymerinjektion, Implantationstherapie) umfasst u.a. die Verwendung von Polymer aus Ethylen-Vinyl-Alkohol. Ein entsprechendes Verfahren wird derzeit mit einem Polymer aus Ethylen-Vinyl-Alkohol (Enteryx^{®}, Boston Scientific, USA) durchgeführt. Es handelt sich dabei um ein synthetisches Polymer, das biologisch nicht abbaubar ist, chemisch inert ist, keine antigenen Eigenschaften aufweist und eine dauerhaft schwammartig-elastische Konsistenz nach Präzipation im Gewebe besitzt. Nach Lösung der Substanz in einem Lösungsmittel (Dimethylsulfoxid) wird das Polymer in flüssigem Zustand über eine endoskopische Injektionskanüle unter radiologischer Kontrolle gezielt in die Ösophaguswand injiziert (Unterstützung der Muskulatur, Druckanhebung). Im Rahmen einer klinischen Studie stellte sich jedoch als nachteilig heraus, dass sich nur bei 60% der Patienten nach 6 Monaten mehr als 50% des injizierten Polymers noch an der Injektionsstelle *in situ* befand, zum Teil waren sogar mehr als 75% der ursprünglich injizierten Menge nicht mehr nachweisbar (Deviere et al., 2002, Endoscopic implantation of a biopolymer in the lower esophageal sphincter for gastro-esophageal reflux: a pilot study. Gastrointes Endsc 2002, 55: 335-41). Offensichtlich kommt es also bei einem beträchtlichen Teil der Patienten im Lauf der Zeit zu einer Abwanderung des Polymers, vermutlich durch die Wand hindurch ins Lumen des Gastrointestinaltraktes hinein. Zudem ist die Verwendung von Dimethylsulfoxid als Lösungsmittel für das verwendete Ethylen-Vinyl-Alkohol Polymer aus gesundheitlichen Gründen als kritisch einzustufen. Aufgrund der technisch vergleichsweise einfachen Methodik und den bisherigen Resultaten erscheint die Biopolymertherapie trotz dieser Ergebnisse zwar weiterhin als attraktiv, allerdings muss die Irreversibilität der Methode (synthetisches, nicht abbaubares Polymer) und die Abwanderung des injizierten Materials als nachteilig kritisch betrachtet werden.

Eine Alternative zu den oben beschriebenen Injektions- und Implantationsverfahren, insbesondere der Biopolymerinjektion, basiert auf der Verwendung von Alginaten. So beschreibt bspw. die WO 2005/105167 die Verwendung von vernetzten Alginaten in Form von implantierbaren Mikrokapseln oder Mikropartikeln oder von Gelen aus mit zwei- oder mehrwertigen Kationen vernetzten Alginaten für die Anwendung als "Filler"-Substanz und in der Behandlung der gastroösophagealen Refluxkrankheit. Auch hier zeigen solche implantierbaren Mikrokapseln oder Mikropartikel keine allergischen Reaktionen oder die Generierung einer körpereigenen Immunantwort des Patienten. Wie zuvor auch für die Faltentherapie beschieben, erfordern die in WO 2005/105167 gezeigten Mikrokapseln oder Mikropartikel, jedoch einerseits deren Bereitstellung vor der Verabreichung in einem separaten Herstellungsschritt, andererseits aber auch einen nicht unerheblichen technischen Aufwand während der Verabreichung. Bevorzugt wäre es daher auch im Bereich der gastroösophagealen Refluxkrankheit, solche Stoffe und Materialien bereitzustellen, die eine einfachere Herstellung und Handhabung ermöglichen.

Eine weitere Erkrankung, die in diesem Zusammenhang von Interesse ist, ist die bereits oben genannte Harninkontinenz. Harninkontinenz, bei der ein unfreiwilliger Harnabgang auftritt, kann als eigenständige Erkrankung oder als Begleiterscheinung zu anderen Erkrankungen vorkommen. Die Harninkontinenz, von der in Deutschland über 6 Millionen Menschen betroffen sind, wird häufig als Tabuthema angesehen, damit verschwiegen und ärztliche Hilfe wird kaum in Anspruch genommen. Daher ist es schwierig, genaue Zahlen über das Auftreten von Harninkontinenz zu erstellen. Schätzungen lassen aber vermuten, dass in Deutschland etwa 11% der über 65-Jährigen und 30% der über 80-Jährigen von der Harninkontinenz betroffen sind. Bei den jüngeren, an Harninkontinenz leidenden Personen überwiegt der Frauenanteil. Der Grund hierfür ist, dass viele Frauen nach der Schwangerschaft und der Geburt eine geschwächte Beckenbodenmuskulatur haben und auf das Training des Beckenbodens nach der Entbindung oft zu wenig Wert legen. Im höheren Lebensalter tritt Harninkontinenz häufig bei Männern als Folge der benignen Prostatahyperplasie auf. Patienten mit Harninkontinenz sind neben dem sozialen Leidensdruck prädisponiert für Harntraktinfektionen, Geschwüre, Ausschläge und Harnsepsis. Allein in den USA werden über 10 Milliarden US Dollar jährlich für den Umgang mit Harninkontinenz ausgegeben.

Die Ursachen für Harninkontinenz können vielfältig sein. Eine Ursache hierfür ist die Muskelschwäche des inneren Schließmuskels (M. sphincter urethrae internus) der Blasenmuskulatur. Bei einer besonderen Form der Harninkontinenz, der vesiko-ureteralen Refluxerkrankung, die häufig bei jüngeren Kindern auftritt, stellt eine weitere Ursache der Rückfluss des Urins durch den Urether von der Blase in Richtung Niere während des Urinlassens dar. Der Urinrückfluss kann dabei durch bakterielle Kontaminationen die Nieren permanent schädigen, von der Narbenbildung bis hin zum Verlust einer oder beider Nieren. Der Weg, Nierenschädigungen zu vermeiden muss in diesem Fall daher sein, Niereninfektionen zu vermeiden. Obwohl der vesiko-ureterale Reflux bei Kindern im Lauf der Zeit wieder von allein vergeht, führt er in einigen Fällen zu schwerwiegenden Harnwegs- und Niereninfektionen bis hin zum Nierenversagen.

Eine Form der Therapie dieser Erkrankungen beruht auf klassischen medikamentösen Behandlungsstrategien. Beispielsweise werden üblicherweise für die Behandlung von Harninkontinenz, insbesondere bei einer aufgetretenen Muskelschwäche des inneren Schließmuskels, weitläufig die Harnblasenmuskulatur relaxierende Substanzen mit anticholinergischen Wirkungen verabreicht (z.B. Wein, 1995, Pharmacology of Incontinence, Urol.clin. North Am., 22: 557-77). Nachteilig wirken sich hierbei aber oft die signifikanten Nebenwirkungen dieser Medikamente aus. Des weiteren kann die v.a. bei Kindern auftretende Form der Harninkontinenz, die vesiko-ureterale Refluxerkrankung durch die langfristige prophylaktische Gabe von Antibiotika behandelt werden. Unglücklicherweise sind aber auch hier solche Behandlungen meist mit unvorhersehbaren Nebenwirkungen verbunden und stellen daher ein nicht kalkulierbares Risiko dar.

Neben der, insbesondere bei Kindern aufgrund der unvorhersehbaren Nebenwirkungen eher unerwünschten, medikamentösen Behandlung werden zur Therapie von Harninkontinenz und insbesondere auch der vesiko-ureteralen Refluxerkrankung auch chirurgische Verfahren eingesetzt, z. B. die Implantation künstlicher Sphinkter (Lima et al., 1996, Combined use of enterocystopasty and a new type of artificial sphincter in the treatment of urinary incontinence, J. Urology, 156: 622-4), Injektion von Kollagenen (Berman et al., 1997, Comparative cost analysis of collagen injection and facia lata sling cystourethropexy for the treatment of type III incontinence in women, J.Urology, 157: 122-4) und Polytetrafluoroethylenen (Perez et al., 1996, Submucosal bladder neck injection of bovine dermal collagen for stress urinary incontinence in the pediatric population, Urology, 156: 633-6). Ähnlich wie bei der Behandlung der gastroösophagealen Refluxkrankheit ("GERD") hat aber die Verwendung von injizierbaren Kollagenen auch hier den nachteiligen Effekt, dass die Behandlung wegen der Abwanderung des Materials häufig wiederholt werden muss (Khullar et al., 1996, GAX Collagen in the treatment of urinary incontinence in elderly women: A two year follow up. British J. Obtetrics & Gynecology, 104: 96-9) und zum Auftreten von Allergien führen kann (McClelland and Delustro, 1996, Evaluation of antibody class in response to bovine collagen treatments in patients with urinary incontinence, J. Urology, 155: 2068-73).

Bei der Therapie der insbesondere bei Kindern auftretenden Form der Harninkontinenz, der vesiko-ureteralen Refluxerkrankung, wird häufig eine chirurgische Korrektion des Refluxes unternommen mit allen bekannten Risken eines chirurgischen Eingriffes. Obwohl sich, wie zuvor dargelegt, der Zustand des vesiko-ureteralen Reflux bei Kindern im Lauf der Zeit bessern oder wieder von allein vergehen kann, führt er in einigen Fällen zu schwerwiegenden Harnwegs- und Niereninfektionen bis hin zum Nierenversagen. Daher besteht insbesondere in diesen Fällen der Bedarf an einer sicheren, effektiven, minimalinvasiven und langfristigen Methode zur Behandlung dieser Refluxerkrankung. Die endoskopische Behandlungsmethode hat dabei gegenüber klassischen chirurgischen Methoden verschiedene Vorteile: sie ist eine ambulante Behandlung, führt zu keiner Narbenbildung, birgt ein geringes Risiko postoperativer Obstruktion und erfordert nur einen geringen Kostenaufwand. Bislang wurden zwar bereits verschiedene Substanzen zur submuskulären Injektion vorgeschlagen (Teflon^{®}, Polydimethylsiloxan^{®}, Macroplastique^{®}, Kollagen (Rind), Zyplast^{®}, autologe Chondrozyten, Fettgewebe und Blut). Zudem wurde das Präparat Deflux^{®} (Dextrantomer/Hyaluronsäure Kopolymer) auch zwischenzeitlich von der FDA zugelassen (Oswald *et al.,* 2002, Prospective comparison and 1-year follow-up of a single endoscopic subureteral polymethylsiloxane versus dextranomer/hyaluronic acid copolymer injection for treatment of vesicoureteral reflux in childeren, Urology 2002; 60: 894-7). Es lässt sich jedoch für alle vorgeschlagenen Anwendungen feststellen, dass die bisherigen Materialien zur Therapie von Harninkontinenz und insbesondere der vesiko-ureteralen Refluxerkrankung den Nachteil haben, dass entzündliche Reaktionen hervorgerufen wurden, die Materialien zum Teil abwanderten oder Mehrfachinjektionen notwendig waren.

Zusammenfassend lässt sich daher feststellen, dass immer noch ein Bedarf an verbesserten körpereigenen oder körperfremden Füllmaterialien besteht, die die oben genannten Nachteile oder besonderen Anforderungen überwinden können und insbesondere bei der Korrektur oder Behandlung von Falten oder Volumendefekten, sowie zur Behandlung von ausgewählten Krankheiten, wie z.B. gastroösophagealer Refluxkrankheit, Harninkontinenz und vesiko-ureteraler Refluxkrankheit, geeignet sind. Eine Lösung der hier gestellten Aufgabe soll insbesondere den folgenden Anforderungen genügen:
1. Das Material soll über sehr dünne Kanülen (>27 Gauge) gut injizierbar sein.
2. Das Material soll am Implantationsort das erwünschte Volumen bilden und langfristig erhalten.
3. Das Implantat soll das gebildete Volumen in seiner geometrischen Form erhalten und behalten.
4. Das Material soll am Injektionsort verbleiben und nicht abwandern.
5. Das Material muss zum Anwendungszeitpunkt und während der *in vivo* Haltbarkeit biokompatibel sein.
6. Das Material soll vorzugsweise weder tierische noch synthetische beziehungsweise nicht abbaubare Bestandteile enthalten.

Diese Aufgabe wird durch die vorliegende Erfindung und die anhängigen Ansprüche gelöst. Insbesondere umfasst die vorliegende Erfindung die Verwendung eines Alginats zur Herstellung einer unvernetzten, hochreinen und hochmolekularen Alginatlösung (Sol) als Füllmaterial in der Medizin, insbesondere Chirurgie, und (der eingreifenden) Kosmetik zum Zweck der Volumenauffüllung, wobei das Alginat in der unvernetzten, hochreinen und hochmolekularen Alginatlösung (Sol) in einer Konzentration insbesondere von etwa 0,5-2,5% (w/v) Alginat Feststoffanteil vorliegt und die unvernetzte, hochreine und hochmolekulare Alginatlösung *in vivo* injiziert wird und zur spontanen Ca²⁺ Vernetzung ohne exogene Zugabe eines Vernetzers führt. Somit besitzt die vorgenannte Alginatlösung die Eignung nach *in vivo* Injektion ohne exogene Zugabe eines Vernetzers vernetzt zu werden.

Im Sinne der vorliegenden Erfindung ist das durch die erfindungsgemäße Verwendung in *situ* gebildete Füllmaterial bevorzugt ein (relativ) festes, hochmolekulares und langzeitstabiles Alginatimplantat, auch monolithisches Alginatimplantat genannt. Ebenfalls bevorzugt ist das gemäß der erfindungsgemäßen Verwendung hergestellte monolithische Alginatimplantat ein Gelkörper. Unter langzeitstabilen monolithischen Alginatimplantaten sind im Sinne der vorliegenden Erfindung bevorzugt ferner solche monolithischen Alginatimplantate zu verstehen, die auch nach (mindestens) 6 Monaten noch am Injektionsort in erheblichem Umfang (mindestens über 50% des Initialvolumens) auftreten.

Im Sinne der vorliegenden Erfindung ist ein Alginat ein natürlich vorkommendes, anionisches, unverzweigtes Polysaccharid, das üblicherweise aus marinen Braunalgen isoliert wird und für dessen Abbau der menschliche Körper - anders als z.B. bei Hyaluronsäure - keine Enzyme hat. Es ist aufgebaut aus homopolymeren Gruppen von beta-D-Mannuronsäure und alpha-L-Guluronsäure, getrennt durch heteropolymere Regionen beider Säuren. Die heute schon in großen Mengen gewonnenen technischen Alginate werden in der Industrie (z.B. Papierherstellung) und als Lebensmittelzusatzstoff (E-Nummern 400-405) eingesetzt (z.B. Askar, 1982, Alginate: Herstellung, Eigenschaften und Verwendung in der Lebensmittelindustrie. Alimenta 21: 165-8). In zunehmenden Maße werden sie jedoch auch in der Pharmazie, Medizin und Biotechnologie eingesetzt. Sie werden routinemäßig als Bestandteil von Wundauflagen verwendet (Gilchrist and Martin, 1983, Wound treatment with Sorbsan - an alginate fibre dressing. Biomaterials 4: 317-20; Agren, 1996, Four alginate dressings in the treatment of partial thickness wounds: A comparative experimental study. Br. J. Plast. Surg. 49: 129-34). Alginate wurden und werden auch in einer ganzen Reihe von "Tissue Engineering" und "Drug Delivery" Projekten eingesetzt (z.B. Uludag et al., 2000, Technology of mammalian cell encapsulation. Advanced Drug Delievery Reviewes 42: 29-64). Die entscheidende Eigenschaft der Alginate für die Verwendung in der Biotechnologie und in der Medizin ist ihre Fähigkeit zur ionotropen Gelbildung. Die Alkalisalze der Alginate sind wasserlöslich, während die Salze der Alginate mit den meisten zwei- oder mehrwertigen Kationen in wässriger Lösung unlösliche Gele (sog. Hydrogele) bilden. Die große physikalische Variationsbreite der Alginate ist durch eine Reihe von Faktoren gegeben: Viskosität (bzw. Molmassenverteilung), Konzentration, Verhältnis der Monomeren und der Affinität des für die Vernetzung typischerweise eingesetzten Kations.

Die Bioverträglichkeit des im Sinne der vorliegenden Erfindung verwendeten Alginats hängt wesentlich von seiner Reinheit ab, vor allem von der Abwesenheit körperfremder Proteine und deren Bruchstücke. Nicht alle Alginate eignen sich daher für die hier beschriebene Verwendung, da sie Verunreinigungen enthalten können, die nach der Implantation in den Menschen Immunabwehrreaktion wie zum Beispiel Fibrose oder entzündliche Reaktionen hervorrufen können (Zimmermann et al., 1992 Production of mitogen-contamination free alginates with variable ratios of mannuronic acid to guluronic acid by free flow electrophoresis, Electrophoresis 13: 269-74). Daher werden bevorzugt solche Alginate verwendet, die keine solchen Verunreinigungen aufweisen, besonders bevorzugt hochreine und hochmolekulare Alginate, und noch stärker bevorzugt hochreines hochmolekulares Kalium- oder Natriumalginat. Im Sinne der vorliegenden Erfindung wird daher eine unvernetzte, in flüssiger Form vorliegende, hochreine und hochmolekulare Alginatlösung (Sol) hergestellt, die bevorzugt zu keinerlei Immunreaktion des angeborenen oder adaptiven Immunsystems eines Patienten führt. Eine solche herzustellende, in flüssiger Form vorliegende, unvernetzte, hochreine und hochmolekulare Alginatlösung (Sol) kann durch die Verwendung homogenen Algenrohmaterials und standardisierter Verfahren (Jork et al., 2000, Biocompatible alginate from freshly collected Laminaria pallida for implantation, Appl. Microbiol Biotechnol 53: 224-229) nach DE 198 36 960 isoliert werden. Dadurch werden die Anforderungen an die Bioverträglichkeit erfüllt.

Das Alginat der erfindungsgemäß hergestellten, unvernetzten, hochreinen und hochmolekularen Alginatlösung (Sol) vernetzt *in situ* nach Verabreichung an einen Patienten, d.h. *in vivo,* durch eine spontan stattfindende Ca²⁺ induzierte Vernetzung derart, dass sich *in vivo,* d.h. nach Verabreichung, vernetzte monolithische Alginatimplantatkörper am Injektionsort bilden. Anders als bislang in der Literatur beschrieben, z.B. in der WO 2005/105167 (Cellmed AG, Deutschland), in der WO 2006/044342 oder in der US 2006/0159823, wurde völlig überraschend erfindungsgemäß festgestellt, dass die im Körper stattfindende spontane Vernetzung ohne exogene Zugabe von zweiwertigen Ionen, wie bspw. Ca²⁺ oder Ba²⁺, zu einem langzeitstabilen Implantat führen kann, wenn hochmolekulares Alginat verwendet wird. WO 2005/105167 lehrt demgegenüber, dass die Langzeitstabilität *in vivo* wesentlich von der kationischen Vernetzung abhängt und dass sich das *ex vivo* durch Zugabe von zweiwertigen Ca²⁺ oder Ba²⁺-ionen vernetzte Gel durch langsamen Kationenaustausch gegen die typischerweise in den natürlichen Geweben und Zellen auftretenden einwertigen Ionen Na⁺oder K⁺ langsam entnetzt und sich somit auflöst. Im Gegenteil dazu konnte nun durch die vorliegende Erfindung gezeigt werden, dass die *in vivo* Stabilität des erfindungsgemäß gebildeten monolithischen Alginatimplantatkörpers vom Molekulargewicht und nicht von der Vernetzung abhängt. Des weiteren wurde, z.B. in der WO 2005/105167, gelehrt, dass bei Injektion von unvernetztem Alginatsol das unvernetzte Alginatsol innerhalb weniger Tage oder Wochen absorbiert und verschwunden ist. Dies trifft in der Tat für niedermolekulare Alginatsole zu, nicht jedoch bei der erfindungsgemäßen Auswahl eines hochmolekularen Alginats. Um möglichst lange, stabile Implantate zu erhalten, werden daher gemäß der vorliegenden Erfindung bevorzugt möglichst hochmolekulare Alginate verwendet.

Im Sinne der vorliegenden Erfindung enthält die in flüssiger Form vorliegende, unvernetzte, hochreine und hochmolekulare Alginatlösung (Sol) daher typischerweise hochreine und hochmolekulare Alginate mit einer mittleren Molmasse von über 200.000 Dalton, bevorzugt mehr als 250.000 Dalton, stärker bevorzugt mehr als 300.000 Dalton, noch stärker bevorzugt mehr als 400.000 Dalton, z.B. bevorzugt hochreine und hochmolekulare Alginate mit einer mittleren Molmasse von 200.000 bis 500.000 Dalton, stärker bevorzugt hochreine und hochmolekulare Alginate mit einer mittleren Molmasse von 200.000 (oder 300.000 oder 400.000) bis 1.000.000 Dalton, und am stärksten bevorzugt hochreine und hochmolekulare Alginate mit einer mittleren Molmasse von 200.000 (oder 300.000 oder 400.000) bis 5.000.000 Dalton.

Das durchschnittliche Molekulargewicht der Alginate kann dabei mit Standardverfahren bestimmt werden, z.B. solchen, wie in Ueno et al., 1988, Chem. Pharm. Bull. 36, 4971-4975; Wyatt, 1993, Anal Chim. Acta; 1-40; and Wyatt Technologies, 1999, "Light scattering University DAWN Course Material" and "DAWN EOS Manual" Wyatt Technology Corporation, Santa Barbara, California, USA, beschrieben.

Die Viskosität einer 0,2 %igen (w/v) (erfindungsgemäß hergestellten, in flüssiger Form vorliegenden, unvernetzten, hochreinen und hochmolekularen) Alginatlösung (Sol) in 0,9% Natriumchloridlösung kann dabei üblicherweise zwischen 3 und 100 mPa s liegen, vorzugsweise liegt sie zwischen 20 und 30 mPa s. Eine solche Wahl der Viskosität ermöglicht insbesondere die Wahl einer sehr dünnen Kanüle zur Injektion, wie im Folgenden beschrieben.

Die Konzentration des Alginates zur Herstellung der erfindungsgemäß hergestellten, unvernetzten, hochreinen und hochmolekularen Alginatlösung (Sol) liegt typischerweise zwischen 0,5 und 2,5% (w/v) Alginat Feststoffanteil und noch stärker bevorzugt in einer Konzentration zwischen 0,6-1,0% (w/v) Alginat Feststoffanteil, bezogen auf das Gesamtgewicht der in flüssiger Form vorliegenden, unvernetzten, hochreinen und hochmolekularen Alginatlösung (Sol). Dabei kann der Anteil der Alginate in der erfindungsgemäß hergestellten, unvernetzten, hochreinen und hochmolekularen Alginatlösung (Sol) mit Verfahren bestimmt werden, die einem Fachmann bekannt sind.

Die erfindungsgemäß hergestellte, unvernetzte, hochreine und hochmolekulare Alginatlösung (Sol) enthält ferner bevorzugt nur so geringe Anteile oder Spuren zweiwertiger Ionen, z.B. eines zweiwertigen Ions aus der Gruppe der Erdalkalimetalle, wie bspw. Mg²⁺, Ca²⁺, Ba²⁺, etc., um eine vorzeitige (d.h. insbesondere spontane) Polymerisation der Alginatlösung (Sol) zu verhindern, besonders bevorzugt keine Anteile oder Spuren solcher zweiwertiger Ionen. Beispielsweise können in der erfindungsgemäß hergestellten, unvernetzten, hochreinen und hochmolekularen Alginatlösung (Sol) bis zu etwa 0,5 mg/l Ca²⁺-lonen (entsprechend etwa 0,00005 % Ca²⁺) und/oder 0,05 mg/l Mg²⁺-lonen (entsprechend etwa 0,000005 % Mg²⁺) enthalten sein, ohne dass eine vorzeitige Polymerisation der Alginatlösung (Sol) eintritt. Mit anderen Worten, vernetzt die erfindungsgemäß hergestellte, unvernetzte hochreine und hochmolekulare Alginatlösung (Sol) *in situ* lediglich aufgrund der physiologisch *in vivo* auftretenden Ca²⁺-lonen oder ggf. anderer physiologisch *in vivo* auftretender zweiwertiger Ionen, und erfordert damit nicht die exogene Zugabe eines Vernetzers. Auch andere zweiwertige Vernetzer treten in der erfindungsgemäß hergestellten, unvernetzten, hochreinen und hochmolekularen Alginatlösung (Sol) bevorzugt nur in einer so geringen Konzentration auf, dass eine Vernetzung der Alginatlösung (Sol) nicht vorzeitig erfolgt. Besonders bevorzugt enthält jedoch die erfindungsgemäß hergestellte, unvernetzte, hochreine und hochmolekulare Alginatlösung (Sol) keine solchen anderen zweiwertigen Vernetzer.

Gemäß einer weiteren Ausführungsform enthält die erfindungsgemäß hergestellte, unvernetzte, hochreine und hochmolekulare Alginatlösung (Sol) auch andere Bestandteile, wie z.B. eine (physiologische) Injektionslösung, Wirkstoffe und/oder weitere Füllstoffe. Die erfindungsgemäß hergestellte, unvernetzte, hochreine und hochmolekulare Alginatlösung (Sol) kann als weiteren Bestandteil auch solche Substanzen enthalten, die das Abwandern des *in situ* gebildeten monolithischen Alginatimplantatkörpers zusätzlich verhindern, z.B. eine bessere Verankerung des monolithischen Alginatimplantatkörpers am Injektions/Transplantationsort ermöglichen. Die jeweiligen Zugaben dieser Bestandteile zur erfindungsgemäß hergestellten, unvernetzten, hochreinen und hochmolekularen Alginatlösung (Sol) kann je nach Erfordernis erst vor Ort durch den behandelnden Arzt erfolgen oder bereits vorher bei der Herstellung der erfindungsgemäß hergestellten, unvernetzten, hochreinen und hochmolekularen Alginatlösung (Sol).

Das Alginat der erfindungsgemäß hergestellten, unvernetzten, hochreinen und hochmolekularen Alginatlösung (Sol) kann in einer (physiologischen) Injektionslösung gelöst sein. Eine solche (physiologische) Injektionslösung umfasst typischerweise jede im Stand der Technik verwendbare Injektionslösung, z.B. eine (physiologische) injizierbare Kochsalzlösung, eine (physiologische injizierbare) Kaliumchlorid-Lösung, eine Lösung, enthaltend Natriumchlorid, Kaliumchlorid und ggf. Natriumacetat, oder ggf. eine Ringer- oder Ringer-Laktat-Lösung, etc., wobei auch diese Lösungen bevorzugt nur so geringe Anteile oder Spuren zweiwertiger Ionen (oder anderer Vernetzer) aufweisen, z.B. zweiwertige Ionen aus der Gruppe der Erdalkalimetalle, wie bspw. Mg²⁺, Ca²⁺, Ba²⁺, etc., um eine vorzeitige Polymerisation der Alginatlösung (Sol) zu verhindern, besonders bevorzugt keine Anteile oder Spuren solcher zweiwertiger Ionen (oder anderer Vernetzer).

Entsprechend einer weiteren Ausführungsform der vorliegenden Erfindung enthält die erfindungsgemäß hergestellte, unvernetzte, hochreine und hochmolekulare Alginatlösung (Sol) solche Substanzen, die das Abwandern des *in situ* gebildeten monolithischen Alginatimplantatkörpers zusätzlich verhindern. Zwar wird bereits durch den *in situ* gebildeten monolithischen Alginatimplantatkörper selbst vorteilhaft eine Diffusion oder ein Abwandern des Füllmaterials in das umliegende Gewebe verhindert. Diese Eigenschaft liegt darin begründet, dass es sich bei dem monolithischen Alginatimplantatkörper einerseits um ein einstückiges, im Vergleich zum umliegenden Gewebe verhältnismäßig großes und unbewegliches Implantat handelt. Andererseits handelt es sich bei dem Implantat zusätzlich um eine weiche, elastische Form, die als solche schlechter im Gewebe verschoben wird, wohingegen harte Formen eher im Gewebe verschoben werden können. Um diesen Effekt noch zu verstärken, können daher der erfindungsgemäß hergestellten, unvernetzten, hochreinen und hochmolekularen Alginatlösung (Sol) auch solche Substanzen beigemengt werden, (die *in situ* bspw. kovalent an das Alginat binden und) die eine weitere histologische Verbindung des *in situ* gebildeten monolithischen Alginatimplantatkörpers nach der Injektion mit dem umgebenen Gewebe bewirken und damit ein Abwandern verhindern. Solche Substanzen umfassen bspw. Adhäsionsproteine (z.B. RGD-Tripeptide).

Gemäß einer weiteren Ausführungsform enthält die erfindungsgemäß hergestellte, unvernetzte, hochreine und hochmolekulare Alginatlösung (Sol) zusätzlich Wirkstoffe, die, ohne darauf beschränkt zu sein, ausgewählt werden aus pharmazeutisch wirksamen Verbindungen, Nährstoffen, Markersubstanzen und vitalen Zellen, z.B. körpereigenen autologen Zellen (des zu behandelnden Patienten), beispielsweise aus Liposuccion. In diesem Zusammenhang können pharmazeutisch wirksame Verbindungen ausgewählt werden aus den Substanzklassen der Vitamine, Adhäsionsproteine, der entzündungshemmenden Substanzen, der Antibiotika, Analgetika, Wachstumsfaktoren, Hormone, besonders bevorzugt ausgewählt aus Protein- und/oder Peptid-basierten Wirkstoffen, wie bspw. humanes Wachstumshormon (human growth hormone), Rinderwachstumshormon (bovine growth hormone), Wachstumshormon aus Schwein (porcine growth hormone), Growth hormone releasing hormone/peptide, Granulozyten-Kolonien stimulierender Faktor (granulocyte-colony stimulating factor), Granulozyten-Makrophagen-Kolonien stimulierender Faktor (granulocyte macrophage-colony stimulating factor), Makrophagen stimulierender Faktor (macrophage-colony stimulating factor), Erythropoietin, Bone morphogenic protein, Interferon oder Derivate davon, Insulin oder Derivate davon, Atriopeptin-III, monoklonale Antikörper, Tumornekrosefaktor, Macrophagen aktivierender Faktor (macrophage activating factor), Interleukin, Tumor degeneriernder Faktor (tumor degenerating factor), Insulin-like growth factor, Epidermaler Wachstumsfaktor (epidermal growth factor), Gewebe-Plasminogen-Aktivator (tissue plasminogen activator), Faktor MV, Faktor IMV, und Urokinase.

Die erfindungsgemäß hergestellte, unvernetzte, hochreine und hochmolekulare Alginatlösung (Sol) kann ferner zusätzlich noch ein wasserlösliches Hilfsmittel enthalten, um die Wirkstoffe zu stabilisieren, z.B. ein Protein, wie bspw. Albumin oder Gelatin; eine Aminosäure, wie bspw. Glycin, Alanin, Prolin, Glutaminsäure, Arginin, oder ein Salz davon; Kohlenhydrate wie bspw. Glucose, Lactose, Xylose, Galaktose, Fruktose, Maltose, Saccharose, Dextran, Mannitol, Sorbitol, Trehalose und Chondroitinsulfat; ein anorganisches Salz wie bspw. Phosphat; ein Benetzungsmittel wie bspw. TWEEN^{®} (ICI), Polyethylenglykol, oder eine Mischung davon.

Die oben genannten Wirkstoffe werden typischerweise während der Herstellung der hier beschriebenen, unvernetzten, hochreinen und hochmolekularen Alginatlösung (Sol) auf im Stande der Technik bekannte Weise eingebracht. Bevorzugt werden diese Wirkstoffe derart ausgewählt, dass die Handhabbarkeit der erfindungsgemäß hergestellten, unvernetzten, hochreinen und hochmolekularen Alginatlösung (Sol) nicht verschlechtert wird (z.B. die Viskosität der Alginatlösung), d.h., dass insbesondere auch Standardkanülen von z.B. 27, 30 oder 33 Gauge, wie hier beschrieben, verwendet werden können.

Entsprechend einer anderen Ausführungsform enthält die erfindungsgemäß hergestellte, unvernetzte, hochreine und hochmolekulare Alginatlösung (Sol) zusätzlich zum Alginat mindestens eine weitere Substanz mit Fülleigenschaften. Wenn die mindestens eine weitere Substanz mit Fülleigenschaft in der unvernetzten, hochreinen und hochmolekularen Alginatlösung (Sol) auftritt, so ist diese mindestens eine weitere Substanz mit Fülleigenschaften bevorzugt in einem Gewichtsanteil von 5-50% vom Filler-Gesamtgewicht (w/w), stärker bevorzugt in einem Gewichtsanteil von 5-40% vom Filler-Gesamtgewicht (w/w), sogar noch stärker bevorzugt in einem Gewichtsanteil von 5-30% vom Filler-Gesamtgewicht (w/w) und am stärksten bevorzugt in einem Gewichtsanteil von 5-20% vom Filler-Gesamtgewicht (w/w) in der Alginatlösung (Sol) suspendiert.

Bevorzugt wird die mindestens eine weitere Substanz mit Fülleigenschaften in der erfindungsgemäß hergestellten, unvernetzten, hochreinen Alginatlösung (Sol), ohne darauf beschränkt zu sein, aus der Gruppe ausgewählt, bestehend aus Hyaluronsäure, Kollagen und Polyacrylamid in löslicher Form. Falls die mindestens eine weitere Substanz mit Fülleigenschaften Hyaluronsäure ist, liegt die Hyaluronsäure bevorzugt als lösliche, unvernetzte, hochgereinigte Hyaluronsäure in einer Konzentration von 5 bis 50%, bezogen auf das Filler-Gesamtgewicht (w/w), in der erfindungsgemäß hergestellten, unvernetzten, hochreinen und hochmolekularen Alginatlösung (Sol) vor.

Im Kontext der vorliegenden Erfindung kann die Hyaluronsäure ausgewählt werden aus Hyaluronsäure oder einem Salz davon, beispielsweise aus Natriumhyaluronat, Kaliumhyaluronat, oder Ammoniumhyaluronat, typischerweise mit einem Molekulargewicht im Bereich von 10.000 Da bis 10.000.000 Da, bevorzugt mit einem Molekulargewicht im Bereich von 25.000 Da bis 5.000.000 Da und am stärksten bevorzugt mit einem Molekulargewicht im Bereich von 50.000 Da bis 3.000.000 Da.

Gemäß einer besonderen Ausführungsform kann die Hyaluronsäure oder ein Salz davon ein Molekulargewicht im Bereich von 300.000 Da bis 3.000.000 Da, bevorzugt ein Molekulargewicht im Bereich von 400.000 Da bis 2.500.000 Da, stärker bevorzugt ein Molekulargewicht im Bereich von 500.000 Da bis 2.000.000 Da, und am stärksten bevorzugt ein Molekulargewicht im Bereich von 600.000 Da bis 1.800.000 Da aufweisen.

Gemäß einer anderen besonderen Ausführungsform kann die Hyaluronsäure oder ein Salz davon ein Molekulargewicht im Bereich von 10.000 Da bis 800.000 Da, bevorzugt ein Molekulargewicht im Bereich von 20.000 Da bis 600.000 Da, stärker bevorzugt ein Molekulargewicht im Bereich von 30.000 Da bis 500.000 Da, noch stärker bevorzugt ein Molekulargewicht im Bereich von 40.000 Da bis 400.000 Da, und am stärksten bevorzugt ein Molekulargewicht im Bereich von 50.000 Da bis 300.000 Da aufweisen.

Der Anteil der Hyaluronsäure oder eines Salzes davon kann mit Verfahren bestimmt werden, die einem Fachmann bekannt sind, z.B. mit Hilfe des Carbazol-Verfahrens (Bitter und Muir, 1962, Anal. Biochem. 4: 330-334). Das durchschnittliche Molekulargewicht der Hyaluronsäure oder einem Salz davon kann ebenfalls mit Standardverfahren bestimmt werden, z.B. solchen, wie in Ueno et al., 1988, Chem. Pharm. Bull. 36, 4971-4975; Wyatt, 1993, Anal Chim. Acta; 1-40; and Wyatt Technologies, 1999, "Light scattering University DAWN Course Material" and "DAWN EOS Manual" Wyatt Technology Corporation, Santa Barbara, California, USA, beschrieben.

Gleichfalls bevorzugt kann die mindestens eine weitere Substanz mit Fülleigenschaften in der erfindungsgemäß hergestellten, unvernetzten, hochreinen und hochmolekularen Alginatlösung (Sol) eine unlösliche Substanz (mit Fülleigenschaften) sein.

Bevorzugt weist diese unlösliche Substanz (mit Fülleigenschaften) eine Partikelgröße zwischen 10 und 150 µm auf. Besonders bevorzugt weisen diese unlöslichen Substanzen eine möglichst runde Partikelform mit einem Durchmesser von 10-80 µm auf. "Möglichst rund" bedeutet im vorliegenden Fall insbesondere eine Form, die im weitesten Sinne einer Kugelform angenähert ist. In diesem Zusammenhang werden in der in der erfindungsgemäß hergestellten, unvernetzten, hochreinen und hochmolekularen Alginatlösung (Sol) unlösliche Substanzen bevorzugt ausgewählt aus der Gruppe bestehend aus Calciumhydroxylapatit, Polymethylmethacylat (PMMA), z.B. als PMMA-Micropartikel, Poly-L-Milchsäure-Mikropartikel, HEMA-Partikel, Calciumhydroxylapatitpartikel, etc..

Ebenfalls bevorzugt sind die oben beschriebenen unlöslichen Substanzen (mit Fülleigenschaften) faserförmig ausgebildet. Bevorzugt weisen diese Stapelfasern einen Durchmesser von 1-80 µm und eine Länge von 10-200 µm auf. Noch bevorzugter weisen diese Stapelfasern einen Durchmesser von 5-40 µm und eine Länge von 20-100 µm auf. Diese Fasern bestehen typischerweise aus gewebeverträglichen, im Körper abbaubaren Polymeren und werden, ohne darauf beschränkt zu sein, bevorzugt ausgewählt aus der Gruppe, bestehend aus Fasern aus Collagen, Polymilchsäure und deren Copolymere (mit Glycin), kovalent vernetzter Hyaluronsäure, Alginsäure, Acryl- und Methacrylsäureesterpolymeren sowie deren Copolymeren.

Alternativ oder zusätzlich kann die mindestens eine weitere Substanz mit Fülleigenschaften in der erfindungsgemäß hergestellten, unvernetzten, hochreinen und hochmolekularen Alginatlösung (Sol) ausgewählt werden aus der Gruppe bestehend aus autologen Bestandteilen, umfassend Zellen, z.B. körpereigene autologe Zellen (des zu behandelnden Patienten), Plasmaproteine oder Liposuccionmaterial, etc..

Die erfindungsgemäß hergestellte, unvernetzte, hochreine und hochmolekulare Alginatlösung (Sol) kann eine oder mehrere der oben genannten Bestandteile enthalten, vorausgesetzt, die Bestandteile sind zusammen biokompatibel, chemisch stabil und zeigen untereinander oder in Bezug auf das enthaltene Alginat bzw. das gebildete monolithische Alginatimplantat keine (störenden) Wechselwirkungen. Insbesondere wird bevorzugt, dass die erfindungsgemäß hergestellte, unvernetzte, hochreine und hochmolekulare Alginatlösung (Sol) nicht bereits vor Verabreichung an einen Patienten aufgrund von einem oder mehreren der oben genannten Bestandteile ganz oder teilweise vernetzt. Auch dürfen die weiteren Bestandteile typischerweise nicht dazu führen, dass der oben als bevorzugt beschriebene Viskositätsbereich der erfindungsgemäß hergestellten, unvernetzten, hochreinen und hochmolekularen Alginatlösung (Sol) verlassen wird.

Die Herstellung und Abfüllung der erfindungsgemäß hergestellten, unvernetzten, hochreinen und hochmolekularen Alginatlösung (Sol) erfolgt üblicherweise steril. Alternativ oder zusätzlich kann die erfindungsgemäß hergestellte, unvernetzte, hochreine und hochmolekulare Alginatlösung (Sol) mit einer geeigneten, dem Stand der Technik entsprechenden Methode endsterilisiert werden, solange keine Verkleinerung des Volumens in erheblichem Umfang stattfindet. Die erfindungsgemäß hergestellte, unvernetzte, hochreine und hochmolekulare Alginatlösung (Sol) ist ferner in gefrorenem Zustand lagerfähig. Die schnelle, unkomplizierte und auch sterile Herstellung der erfindungsgemäß hergestellten, unvernetzten, hochreinen und hochmolekularen Alginatlösung (Sol) ist dabei ein wesentlicher Vorteil der vorliegenden Erfindung gegenüber den im Stand der Technik gezeigten Materialien, insbesondere der Herstellung von Beads/Partikeln.

Gemäß einer besonderen Ausführungsform der vorliegenden Erfindung umfasst die Erfindung die Verwendung eines Alginats zur Herstellung einer wie hier beschriebenen, unvernetzten, hochreinen und hochmolekularen Alginatlösung (Sol) zur Behandlung von Hautfalten, v.a. im Bereich des Gesichts, z.B. im Bereich der Mimikmuskulatur, sowie des Dekolleté, der Hände; zur Behandlung von Volumendefiziten, insbesondere zur Volumenauffüllung z.B. bei Lipoatrophie, der Brüste, z.B. nach der Operation von Mammakarzinomen oder zur Brustvergrößerung, etc.; und insbesondere zu kosmetischen Zwecken, oder zur Behandlung von ausgewählten Krankheiten, wie z.B. gastroösophagealer Refluxkrankheit, Harninkontinenz oder vesiko-ureteraler Refluxkrankheit, bspw. durch Unterstützung von Sphinktermuskulaturen durch Unterspritzung in die entsprechende Sphinktermuskulatur, zur Verwendung in der Wiederherstellungschirurgie, oder in der Tumortherapie.

Die vorliegende Erfindung beschreibt u.a. auch die Verwendung eines Alginats zur Herstellung einer unvernetzten, hochreinen und hochmolekularen Alginatlösung (Sol) als Füllmaterial in der Medizin, insbesondere der (dermatologischen) Chirurgie und Kosmetik (bspw. zum Zweck der Volumenauffüllung), wobei das Alginat in der unvernetzten, hochreinen und hochmolekularen Alginatlösung (Sol) in einer Konzentration von etwa 0,5-2,5% (w/v) Alginat Feststoffanteil vorliegt und die unvernetzte, hochreine und hochmolekulare Alginatlösung *in vivo* injiziert wird und zur spontanen *in situ* Ca²⁺ Vernetzung ohne exogene Zugabe eines Vernetzers führt. Insbesondere beschreibt die vorliegende Erfindung die Verwendung dieser unvernetzten, hochreinen und hochmolekularen Alginatlösung (Sol) zur Behandlung von Hautfalten, v.a. im Bereich des Gesichts, z.B. im Bereich der Mimikmuskulatur, sowie des Dekolleté, der Hände; zur Behandlung von Volumendefiziten, insbesondere zur Volumenauffüllung z.B. bei Lipoatrophie, der Brüste, z.B. nach der Operation von Mammakarzinomen oder zur Brustvergrößerung, etc.; und insbesondere zu kosmetischen Zwecken, oder zur Behandlung von ausgewählten Krankheiten, wie z.B. gastroösophagealer Refluxkrankheit, Harninkontinenz oder vesiko-ureteraler Refluxkrankheit, bspw. durch Unterstützung von Sphinktermuskulaturen durch Unterspritzung in die entsprechende Sphinktermuskulatur, zur Verwendung in der Wiederherstellungschirurgie, in der Tumortherapie.

Die erfindungsgemäß hergestellte, unvernetzte, hochreine und hochmolekulare Alginatlösung (Sol) wird dabei typischerweise in flüssiger Form mittels Injektion an den Patienten verabreicht. Bevorzugt erfolgt die Verabreichung der erfindungsgemäß hergestellten, unvernetzten, hochreinen und hochmolekularen Alginatlösung (Sol) durch transdermale, intradermale, subdermale, subkutane oder intramuskuläre Injektion in eine geeignete Injektionsstelle des Patienten. Die Wahl der Injektionsstelle und das zu verabreichende Injektionsvolumen hängen dabei von dem zu behandelnden Zustand oder der zu therapierenden Krankheit ab, besonders bevorzugt solchen zu behandelnden Zuständen oder zu therapierenden Krankheiten, wie hier beschrieben.

Die Verabreichung der erfindungsgemäß hergestellten, unvernetzten, hochreinen und hochmolekularen Alginatlösung (Sol) erfolgt dabei üblicherweise durch Injektion mit Kanülen mit einem Durchmesser von typischerweise 20 bis 33 Gauge, bevorzugt von 26 bis 33 Gauge. Bevorzugt sollte daher die erfindungsgemäß hergestellte, unvernetzte, hochreine und hochmolekulare Alginatlösung (Sol) mit einer Spritze, die eine Kanüle der vorgenannten Art aufweist, verabreichbar sein. Besonders bevorzugt sind handelsübliche Kanülen, z.B. mit einem Durchmesser von 27 bis 33 Gauge. Alternativ kann die erfindungsgemäß hergestellte, unvernetzte, hochreine und hochmolekulare Alginatlösung (Sol) mittels geeigneter anderer Techniken, die im Stand der Technik bekannt sind, verabreicht werden, z.B. durch Verwendung endoskopischer oder laparoskopischer Techniken. Die Injektion kann entweder durch einmaliges, mehrmaliges bzw. vielfaches Einspritzen in die gleichen oder in verschiedene (typischerweise benachbarte) Areale, z.B. der Haut oder der Sphinktermuskulatur, erfolgen.

Das zu verabreichende Injektionsvolumen der erfindungsgemäß hergestellten, unvernetzten, hochreinen und hochmolekularen Alginatlösung (Sol) liegt dabei üblicherweise in einem Bereich von 0,1 bis 100 ml, bevorzugt in einem Bereich von 0,1 bis 50 ml, stärker bevorzugt in einem Bereich von 0,1 bis 40 ml, noch stärker bevorzugt in einem Bereich von 0,1 bis 30, 20 oder 10 ml, und am stärksten bevorzugt in einem Bereich von 0,1 bis 1, 2 oder 5 ml. Ebenfalls kann die Menge des zu verabreichenden Injektionsvolumens auch über 100 ml liegen, falls z.B. große Volumendefizite aufgefüllt werden müssen. Voraussetzung für die Wahl des zu verabreichenden Injektionsvolumens ist typischerweise, dass das zu behandelnde Gewebe derartig durchblutet und/oder mit Ca²⁺-lonen versorgt ist, dass eine Vernetzung der erfindungsgemäß hergestellten, unvernetzten, hochreinen und hochmolekularen Alginatlösung (Sol) *in situ,* d.h. im Gewebe, innerhalb eines begrenzten Zeitraumes, z.B. eines Zeitraumes von maximal mehreren Stunden (z.B. 0-24 Stunden, bevorzugt 0-10 Stunden, stärker bevorzugt 0-1, 2 oder 5 Stunden) erfolgen kann. Dabei kann der zu behandelnde Arzt durch geeignete Mittel für einen im Vorfeld der Therapie, parallel dazu oder in der Nachbehandlung ausgeglichenen Ca²⁺-Haushalt des Patienten sorgen. Bevorzugt umfassen solche Verfahren eben nicht die im Stand der Technik beschriebene Co-Administration von Vernetzer und Alginatlösung (Sol) sondern nutzen ggf. andere Verfahren, wie z.B. eine entsprechende Diät, der oralen Aufnahme calciumreicher Produkte, etc.. Die Wahl des zu verabreichenden Injektionsvolumens hängt dabei auch von der Beurteilung des zu behandelnden Arztes ab. So wird von der erfindungsgemäß hergestellten, unvernetzten, hochreinen und hochmolekularen Alginatlösung (Sol) typischerweise eine sichere und effektive Menge verabreicht. Wie hier verwendet bedeutet "sichere und effektive Menge" eine solche Menge der erfindungsgemäß hergestellten, unvernetzten, hochreinen und hochmolekularen Alginatlösung (Sol), die ausreichend ist, um signifikant eine Veränderung des zu behandelnden Zustandes oder der zu behandelnden Krankheit zu bewirken, jedoch gering genug, um schwerwiegende Nebeneffekte zu vermeiden (mit einem vernünftigen Verhältnis von Vorteil/Risiko), d.h. innerhalb des Bereichs vernünftiger medizinischer Urteilskraft. Eine sichere und effektive Menge der erfindungsgemäß hergestellten, unvernetzten, hochreinen und hochmolekularen Alginatlösung (Sol) wird daher typischerweise im Zusammenhang mit dem besonderen zu behandelnden Zustand oder der zu behandelnden Krankheit variieren, sowie dem Alter und dem physischen Zustand des zu behandelnden Patienten, der Schwere des Zustandes, der Dauer der Behandlung, der Natur einer ggf. begleitenden Therapie, und ähnlichen Faktoren innerhalb des Wissens und der Erfahrung des begleitenden Arztes. Die erfindungsgemäß hergestellte, unvernetzte, hochreine und hochmolekulare Alginatlösung (Sol) kann für humane und auch für veterinärmedizinische Zwecke eingesetzt werden.

Gemäß einer ersten Alternative wird die erfindungsgemäß hergestellte, unvernetzte, hochreine und hochmolekulare Alginatlösung (Sol) zur Behandlung von Hautfalten eingesetzt. Die Behandlung von Hautfalten umfasst dabei üblicherweise die Behandlung von solchen Hautdefiziten, die durch z.B. Altern, Umwelteinflüsse, Gewichtsverlust, Schwangerschaft, Krankheiten, insbesondere HIV-Infektion, chirurgische Eingriffe und Akne verursacht werden. Insbesondere umfasst die Behandlung von Hautfalten die Behandlung von Gesichtsfalten, insbesondere im Bereich der Mimikmuskulatur, die Behandlung von Stirnfalten, Zornesfalten, Kummerfalten, Schlupflidern, Krähenfüßen, Nasolabialfalten, die Verwendung zur Lippenunterspritzung sowie die Behandlung von Falten im Handbereich und im Dekolleté. Die Behandlung erfolgt typischerweise durch intra- oder subdermale Unterspritzung der betroffenen Hautareale. Vorzugsweise wird dazu die erfindungsgemäß hergestellte, unvernetzte, hochreine Alginatlösung (Sol) durch eine Spritze mit einem Kanülendurchmesser von 20 bis 33 Gauge, stärker bevorzugt von 26 bis 33 Gauge, injiziert. Alternativ kann die erfindungsgemäß hergestellte, unvernetzte, hochreine Alginatlösung (Sol) mittels geeigneter anderer Techniken, die im Stand der Technik bekannt sind, verabreicht werden. Die Injektion kann entweder durch einmaliges, mehrmaliges bzw. vielfaches Einspritzen in die gleichen oder in verschiedene (z.B. benachbarte) Hautareale erfolgen. Bei der Behandlung von Hautfalten wird bei jedem Einstich bevorzugt nur ein kleines Volumen übertragen, stärker bevorzugt in einem Bereich von 0,1 ml bis 10 ml, noch stärker bevorzugt in einem Bereich von 0,1 ml bis 5, 2 oder nur 1 ml, bis das gewünschte Gesamtvolumen unterspritzt wurde. Dadurch wird eine flächige Aufpolsterung und eine Straffung der Haut erzielt, die zum Verschwinden oder teilweisen Verschwinden der Falten und/oder Volumendefizite in dem entsprechenden Areal führt. Die Injektion kann dabei auch einmalig, mehrmalig bzw. vielfach derart erfolgen, dass im Ergebnis ein Volumen von 0,1 ml bis 10 ml oder sogar mehr appliziert wird. Die Applikation erfolgt dabei, üblicherweise bei jeder einzelnen Verabreichung, vorzugsweise durch langsames Zurückziehen der Injektionskanüle/-nadel bei gleichzeitiger Volumenunterspritzung. Diese Injektionsmethode eignet sich insbesondere bei tieferen Falten. Die hier beschriebene erfindungsgemäße Anwendung zur Faltenunterspritzung kann jederzeit mit konventionellen Behandlungsmethoden kombiniert werden. Solche Behandlungsmethoden schließen bspw. klassische chirurgische und/oder medikamentöse Behandlungsmethoden ein.

Gemäß einer zweiten Alternative wird die erfindungsgemäß hergestellte, unvernetzte, hochreine und hochmolekulare Alginatlösung (Sol) zur Behandlung der gastroösophagealen Refluxkrankheit eingesetzt. Typischerweise erfolgt die Behandlung im Sinne der vorliegenden Erfindung durch Injektion der erfindungsgemäß hergestellten, unvernetzten, hochreinen und hochmolekularen Alginatlösung (Sol) in die Wandbereiche des unteren ösophagealen Sphinkter-Muskels bzw. das umgebende Gewebe. Das Sphinkter Volumen erhöht sich dabei proportional zum Volumen der injizierten, erfindungsgemäß hergestellten, unvernetzten, hochreinen und hochmolekularen Alginatlösung (Sol). Dadurch wird das innere Lumen des Sphinktermuskels verkleinert und erlaubt somit eine bessere Kontraktion des Muskels und verhindert somit ein Austreten der Magensäure in den Ösophagus. Die Inkjektion ist vorzugsweise mit Standardtechniken durchzuführen die dem Stand der Technik entsprechen, wie z.B. direkte Injektionen oder durch Verwendung endoskopischer oder laparoskopischer Techniken. Auch die hier beschriebene erfindungsgemäße Anwendung zur Behandlung der gastroösophagealen Refluxkrankheit kann mit konventionellen Behandlungsmethoden kombiniert werden. Solche Behandlungsmethoden schließen bspw. klassische chirurgische und/oder medikamentöse Behandlungsmethoden ein.

Gemäß einer dritten Alternative kann die erfindungsgemäß hergestellte, unvernetzte, hochreine und hochmolekulare Alginatlösung (Sol) zur Behandlung der Harninkontinenz und der vesiko-ureteralen Refluxkrankheit eingesetzt werden. Die Verwendung der erfindungsgemäß hergestellten, unvernetzten, hochreinen Alginatlösung (Sol) eignet sich auch bei temporären, nicht chronischem Auftreten von Formen der Harninkontinenz sowie der vesiko-ureteralen Refluxkrankheit. Die Behandlung dieser Erkrankungen erfolgt typischerweise durch Injektion der erfindungsgemäß hergestellten, unvernetzten, hochreinen Alginatlösung (Sol) in den ureteralen Sphinkter, den Blasensphinkter oder die Harnröhrenmuskulatur. Das Sphinkter-Volumen erhöht sich dabei proportional zum Volumen der injizierten erfindungsgemäß hergestellten, unvernetzten, hochreinen Alginatlösung (Sol). Dadurch wird auch hier das innere Lumen des Sphinktermuskels verkleinert und erlaubt somit eine bessere Kontraktion des Muskels wodurch die Wahrscheinlichkeit einer Harninkontinenz sinkt. Die Inkjektion ist auch hier vorzugsweise mit Standardtechniken durchzuführen, die dem Stand der Technik entsprechen, wie z.B. direkte Injektionen oder durch Verwendung endoskopischer oder laparoskopischer Techniken. Die hier beschriebene erfindungsgemäße Anwendung zur Behandlung der Harninkontinenz und der vesiko-ureteralen Refluxkrankheit kann ebenfalls mit konventionellen Behandlungsmethoden kombiniert werden. Solche Behandlungsmethoden schließen bspw. klassische chirurgische und/oder medikamentöse Behandlungsmethoden ein.

Gemäß einer vierten Alternative kann die erfindungsgemäß hergestellte, unvernetzte, hochreine und hochmolekulare Alginatlösung (Sol) in der Wiederherstellungschirurgie und insbesondere zu kosmetischen Zwecken eingesetzt werden. Solche Fälle umfassen z.B. Fälle, in denen ein Volumendefekt oder Volumendefizit im Gewebe auftritt, z.B. falls Tumorgewebe operativ entnommen wurde oder Gewebe fehlt und dadurch eine Kavität, d.h. ein Hohlraum im Gewebe, verbleibt, der auffüllungsbedürftig ist und/oder aus kosmetischen Gründen kaschiert werden soll. Ebenfalls umfasst sind hier solche Fälle, in denen nach einem Unfall, einem Eingriff oder einer Erkrankung wie z.B. HIV, etc., die Notwendigkeit zur strukturellen (kosmetischen) Wiederherstellung des betroffenen Körperteils oder Gewebes besteht.

Gemäß einer fünften Alternative kann die erfindungsgemäß hergestellte, unvernetzte, hochreine und hochmolekulare Alginatlösung (Sol) daher auch zur Behandlung der facialen (HIV-induzierten) Lipodystrophie, insbesondere zur Behandlung der (HIV-induzierten) Lipoatrophie eingesetzt werden. Im Zusammenhang mit der vorliegenden Erfindung ist (HIV-induzierte) Lipoatrophie eine Erkrankung, die v.a. durch Fettgewebeschwund mit Rückbildung von Fettgewebe gekennzeichnet ist, z.B. aufgrund der Behandlung von HIVinfizierten Patienten mit Nukleosid-Inhibitoren der reverse Transkriptase (NRTIs). Pathologisch macht sich diese Erkrankung insbesondere bei HIV-Infektion im Bereich der Extremitäten, d.h. Armen und/oder Beinen, aber auch des Gesäßes und der Wangen bemerkbar und führt bei den Patienten aufgrund der rapiden und teilweise erschreckenden äußerlichen Veränderung oftmals zu psychischen Störungen und in der Folge zur gesellschaftlichen Abgrenzung. Die Behandlung der (HIV-induzierten) Lipoatrophie kann durch Unterspritzung der entsprechenden Hautpartien, v.a. im Gesichtsbereich, erfolgen, z.B. im Bereich des Zygomaticus major, etc.. Die Wahl des Injektionsortes und die Menge der zu verabreichenden, erfindungsgemäß hergestellten, unvernetzten, hochreinen und hochmolekularen Alginatlösung (Sol) hängt dabei von der Schwere der Erkrankung ab, und liegt typischerweise in der Beurteilung des zu behandelnden Arztes, bevorzugt innerhalb der oben genannten Werte.

Gemäß einer weiteren letzten Alternative kann die erfindungsgemäß hergestellte, unvernetzte, hochreine und hochmolekulare Alginatlösung (Sol) auch zur Behandlung von Tumorerkrankungen, z.B. durch Embolisierung des Tumors, eingesetzt werden. Dabei können insbesondere die tumorspezifisch gebildeten Gefäße, die die Versorgung des Tumors sicherstellen sollen, z.B. neugebildete Arterien, durch Injektion der erfindungsgemäß hergestellten, unvernetzten, hochreinen und hochmolekularen Alginatlösung (Sol) in diese Gefäße blockiert werden, wodurch die Tumorzellen von der Versorgung abgeschnitten werden und absterben. In diesem Zusammenhang werden Tumorerkrankungen, ohne darauf beschränkt zu sein, bspw. ausgewählt aus der Gruppe, bestehend aus Melanomen, malignen Melanomen, Kolon-Karzinomen, Lymphomen, Sarkomen, Blastomen, Nierenkarzinomen, Gastrointestinalen Tumoren, Gliomen, Prostatatumoren, Blasenkrebs, Rektaltumoren, Magenkrebs, Speiseröhrenkrebs, Bauchspeicheldrüsenkrebs, Leberkrebs, Mammakarzinomen (=Brustkrebs), Gebärmutterkrebs, Gebärmuttehalskrebs, Hepatomen, diversen Virus-induzierten Tumoren, wie z.B. Papillomvirus-induzierten Karzinomen (z.B. Cervixkarzinom = Gebärmutterhalskrebs), Adenokarzinomen, Herpesviren-induzierten Tumoren (z.B. Burkitt's Lymphom, EBV-induziertem B Zelllymphom), Hepatitis B-induzierten Tumoren (Hepatozellkarzinomen), HTLV-1- und HTLV-2-induzierten Lymphomen, Akustikusneurinom, Lungenkarzinomen (= Lungenkrebs = Bronchialkarzinom), kleinzelligen Lungenkarzinomen, Rachenkrebs, Analkarzinom, Glioblastom, Rektumkarzinom, Astrozytom, Hirntumoren, Retinoblastom, Basaliom, Medulloblastomen, Scheidenkrebs, Hodenkrebs, Schilddrüsenkarzinom, Hodgkin-Syndrom, Meningeomen, Schneeberger Krankheit, Hypophysentumor, Karzinoiden, Neurinom, Spinaliom, Burkitt-Lymphom, Kehlkopfkrebs, Nierenkrebs, Thymom, Corpuskarzinom, Knochenkrebs, Non-Hodgkin-Lymphomen, Urethrakrebs, Kopf-Hals-Tumoren, Oligodendrogliom, Vulvakrebs, Darmkrebs, Kolonkarzinom, Ösphaguskarzinom (= Speiseröhrenkrebs), Warzenbeteiligung, Dünndarmtumoren, Kraniopharyngeomen, Ovarial-Karzinom, Weichteiltumoren, Eierstockkrebs (= Ovarialkarzinom), Pankreaskarzinom (=Bauchspeicheldrüsenkrebs), Endometriumkarzinom, Lebermetastasen, Peniskrebs, Zungenkrebs, Gallenblasenkrebs, Leukämie, Plasmozytom, Lidtumor, Prostatakrebs (= Prostatatumoren), etc..

Vorteilhafterweise ist in allen zuvor genannten Anwendungen und Alternativen das durch Injektion der erfindungsgemäß hergestellten und verwendeten unvernetzten, hochreinen und hochmolekularen Alginatlösung (Sol) *in situ* erzeugte monolithische Alginatimplantat biologisch verträglich und bietet einen guten Haftgrund zur Bewachsung mit Zellen oder Kollagenfasern. Sollte dennoch eine Unverträglichkeit im Einzelfall gegeben sein, kann das gebildete monolithische Alginatimplantat ggf. durch Injektion einer EDTA- oder Citratlösung, oder einer Lösung aus anderen Komplexbildnern, bevorzugt direkt in das gebildete monolithische Alginatimplantat, wieder aufgelöst werden.

Abschließend stellt die vorliegende Erfindung auch Kits bereit, umfassend die erfindungsgemäß hergestellte, unvernetzte, hochreine Alginatlösung (Sol), ggf. eine oder mehrere der hier beschriebenen Injektionskanülen zur Verabreichung, und ggf. eine Gebrauchsanleitung.

### Vorteile der Erfindung

Die Verwendung eines Alginats zur Herstellung einer wie hier beschrieben, unvernetzten, hochreinen und hochmolekularen Alginatlösung (Sol) als Füllmaterial in der Medizin, insbesondere Chirurgie, und Kosmetik zum Zweck der Volumenauffüllung zeigt gegenüber dem bisherigen Stand der Technik erhebliche Vorteile. Die primären Vorteile der vorliegenden Erfindung lassen sich wie folgt zusammenfassen:
a) Einfache Injektion der flüssigen erfindungsgemäß hergestellten, unvernetzten, hochreinen und hochmolekularen Alginatlösung (Sol) bei geringem Injektionsdruck.
   Wesentliche Vorteile gegenüber einer Coinjektion von Alginatsol und Ca²⁺- oder Ba²⁺-salz sind insbesondere:
   - Die Verwendbarkeit von Standard- Injektionskanülen/-nadeln bis zu 33 Gauge.
   - Direkte Verwendung einer einfachen Fertigspritze ohne Doppelkammer, Mischkammer oder Doppelkanüle.
   - Dadurch einfachere, exaktere Handhabung bezüglich Injektionsort und -volumen.
   - Keinerlei Beeinträchtigung der Injektionstätigkeit durch vorzeitige Vernetzung in der Nadel.
   - Eine einfache Möglichkeit der Zumischung von anderen Polymeren, Füllstoffen, Wirkstoffen und vor allem Zellen, insbesondere autologen Zellen, vor Ort.
   Wesentliche Vorteile gegenüber der Injektion von Beadpräparationen sind insbesondere:
   - Die viel einfachere und v.a. billigere aseptische Herstellung der Alginatlösung (Sol) gegenüber der Herstellung von Beads oder Partikeln.
   - Eine bessere Formgebung des Implatatkörpers, da dieser monolithisch in Form des Injektionsvolumens vorliegt.
   - Wegfall der Produktionsschritte Beadbildung, Vernetzung im Vernetzerbad, Waschschritt danach.
   - Einfache Zumischung von Drittstoffen (siehe oben).
b) *In situ* Bildung eines stabilen, vernetzten, monolithischen AlginatImplantatkörpers genau der gewünschten Geometrie entsprechend, welcher während und kurz nach der Injektion geformt werden kann.
c) Bildung des stabilen Implantatvolumens entsprechend dem Injektionsvolumen ohne Volumenschwund und der Notwendigkeit der Nachkorrektur.
d) Bildung langfristig stabiler Implantate mit Haltbarkeiten von (deutlich) mehr als 6 Monaten bei entsprechender Wahl des Alginats.
e) Zugabe von anderen löslichen Wirkstoffen oder Polymeren wie Hyaluronsäure, Kollagen und ähnliches ist leicht möglich.
f) Zugabe von festen, unlöslichen Partikeln oder Mikrofasern unterschiedlicher *in vivo* Stabilität wie Hydroxylapatit, Polymethylmethacylat (PMMA), Poly-L-Milchsäure-Mikropartikeln ist ebenfalls leicht möglich.
g) Auch die Zugabe von autologen Zellen, Proteinen, Fett etc. ist leicht möglich, auch durch den behandelnden Arzt vor Ort.

Darüber hinaus und von größter Bedeutung ist das weit überlegene klinische Ergebnis. Im Gegensatz zu Bead-Implantaten kann bei den erfindungsgemäßen Alginatsolinjektionen ein langsamer Ersatz des Implantatvolumens durch das Einwachsen von Kollagenfasern (siehe Experimenteller Teil) erfolgen. Damit ist das monolithische Alginatimplantat nicht nur in hohem Maße gewebeverträglich gegenüber dem umliegenden Gewebe, sondern durch seine hohe Durchlässigkeit und Verträglichkeit auch ein ausgezeichneter Platzhalter für Zellen. Die lange Haltbarkeit des Implantatkörpers gewährt dabei auch für langsam wachsendes Gewebe den entsprechenden Platzhalter.

### Figuren

Die folgenden Figuren sollen die vorliegende Erfindung lediglich beispielhaft erläutern, aber in keiner Weise einschränken.

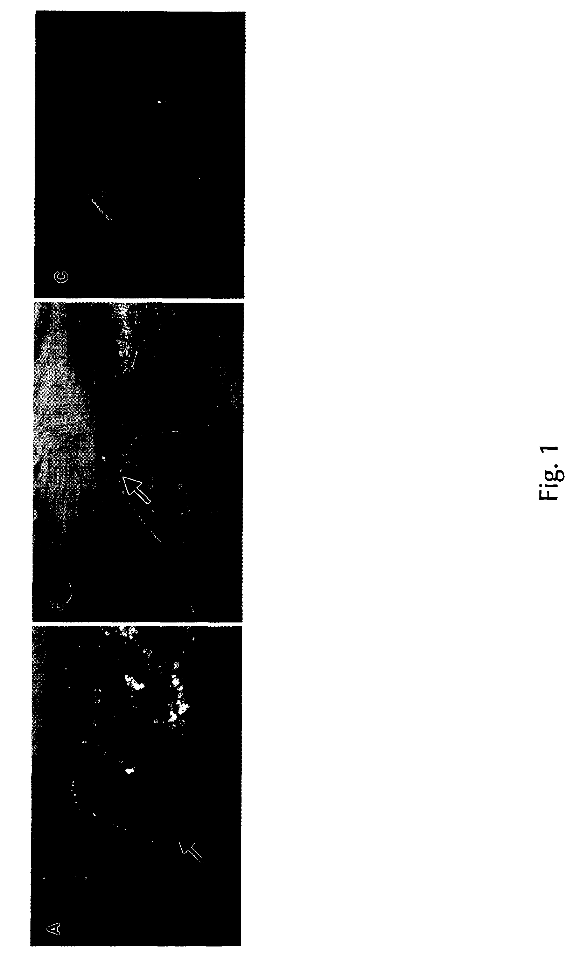

- Figur 1:: stellt die Explantation nach Verabreichung der Prüfsubstanz 1 (unvernetzte Alginatlösung) 60 Minuten nach Applikation dar.
A: zeigt die subkutan injizierte Alginatlösung, die sich auch nach der Freipräparation der Haut makroskopisch als Schwellung darstellt,
B: zeigt, dass die applizierte Prüfsubstanz gut tastbar und formstabil ist,
C: belegt, dass die ehemals flüssige Alginatlösung bereits 60 Minuten nach Applikation durch endogenes Calcium vernetzt und als Gelkissen explantierbar ist.
- Figur 2:: zeigt H&E Färbungen der Stellen der Injektion der erfindungsgemäß hergestellten, unvernetzten, hochreinen Alginatlösung (Sol) 6 Monate nach der subkutanen Injektion in die Ratte.
A: stellt die Übersicht über ein Alginat-Depot dar.
B: zeigt ein Detail eines Alginat-Depots innerhalb des Fettgewebes mit eingebetteten Kollagenfasern.
- Figur 3:: zeigt Kollagenfärbungen (van Gieson Färbung) der Injektionsstellen mit den Testimplantaten 1 (AL-018; A, B) und 2 (AL-019; C, D) vier Wochen nach der Injektion in das Kaninchen.
A: zeigt Kollagenfasern innerhalb des i.d. injizierten Testimplantats 1; die Kollagenfasern sind gleichmäßig rubinrot gefärbt wie die ursprünglichen dermalen Kollagenbündel;
B: stellt eine detaillierte Mikroaufnahme des s.c. injizierten Testimplantats 1 dar, das mit Kollagenfasern durchsetzt ist;
C: zeigt ein s.c. injiziertes Testimplantat 2, das mit einer Vielzahl von Kollagenfasern durchsetzt ist;
D: beschreibt eine detaillierte Mikroaufnahme des s.c. injizierten Testimplantats 2, das mit Kollagenfasern verschiedener Länge durchsetzt ist.

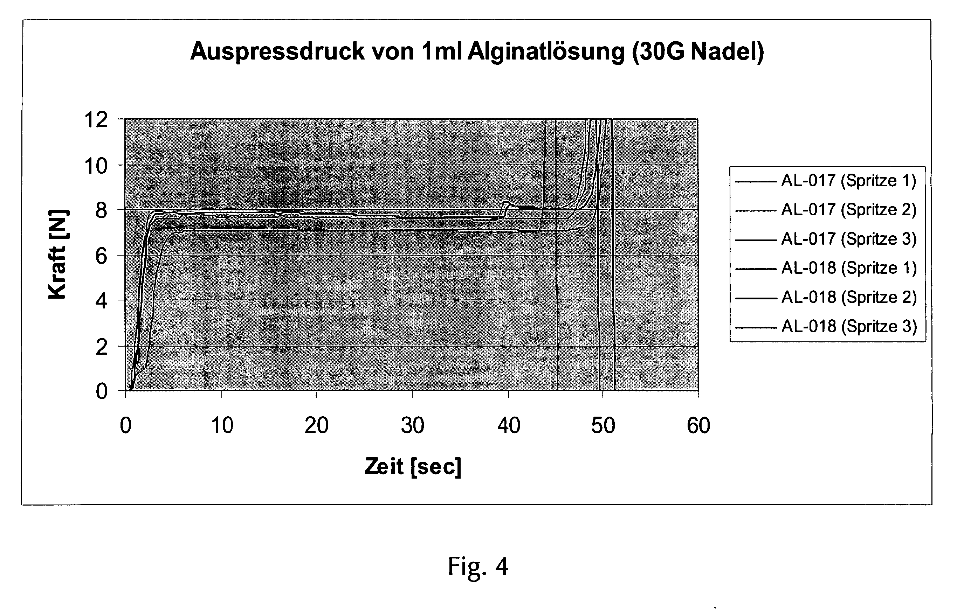

- Figur 4:: zeigt die Darstellung des Auspressdruckes von 1 ml Alginatlösung mittels einer 30G Kanüle. Wie leicht zu erkennen ergibt sich über den gesamten Bereich (mit Ausnahme des Beginns und des Endes aufgrund der physikalischen Gegebenheiten) ein gleichmäßiger Druck, d.h. die erfindungsgemäß hergestellte, unvernetzte, hochreine Alginatlösung (Sol) lässt sich mit einem verhältnismäßig geringem Druck, also verhältnismäßig leicht, aus einer 30G Kanüle pressen.

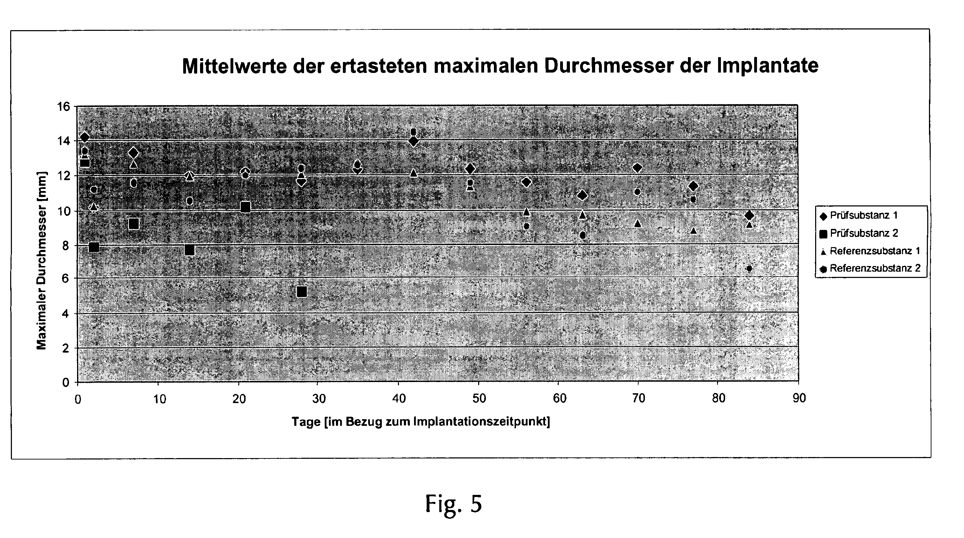

- Figur 5:: zeigt die Darstellung der Mittelwerte der ertasteten Maximal- und Minimaldurchmesser der verschiedenen Test-Implantate (erzeugt mittels Injektion der Prüfsubstanzen bzw. Implantation der Referenzsubstanzen) im Versuchszeitraum. Das Implantat wurde am narkotisierten Tier (Ratte) ertastet und die Implantatgröße mit Hilfe einer Schieblehre vermessen.
Prüfsubstanz 1 (unvernetzte hochmolekulare Alginatlösung);
Prüfsubstanz 2 (niedermolekulare unvernetzte Alginatlösung),
Referenzsubstanz 1 (CellBeads^{®} 500),
Referenzsubstanz 2 (CellBeads^{®} 500, autoklaviert).
Die Daten resultieren aus dem Vermessen folgender Implantatstellen: Tag 1 n=16, Tag 2-7 je n=12, Tag 14 -28 je n=8, verbleibende Tage je n=4.

### Beispiele

Die folgenden Beispiele sollen die vorliegende Erfindung lediglich beispielhaft erläutern, aber in keiner Weise einschränken.

### 1. Herstellung der 1% (w/v) Alginatlösung

0,25 g getrocknetes, hochreines Alginat (Reinheit >99 %, MW > 500.000 g/mol) wurde unter Reinraumbedingungen in 25 ml 0,9 % NaCl-Lösung eingetragen. Das verschlossene Röhrchen wurde anschließend bei Raumtemperatur bis zur vollständigen Auflösung des Na-Alginats rotiert. Diese Lösung wurde nach nochmaliger Sterilfiltration (0,2 µm Spritzenvorsatzfilter) in 1 ml Spritzen zu je 500 µl abgefüllt und das Fertigprodukt (Spritzen) wurde bei 5°C plus/minus 3°C gelagert. Für die Injektion wurde eine Nadel von 30G verwendet.

### 2. Herstellung von Alginatkapseln (Vergleichsversuch)

Die Alginatlösung wurde, wie in Beispiel 1 beschrieben hergestellt, wobei nur 0,15 g desselben Alginats in 25 ml NaCl Lösung eingetragen wurden. Zur vollständigen Lösung wurde wieder im geschlossenen Gefäß auf einem Reagenzglasrotationsgerät bis zur vollständigen Lösung rotiert.

Diese Lösung wurde nach Sterilfiltration (0,2 µm Filter) über eine handelsübliche Vertropfungsapparatur unter Kontrolle der Tropfengröße in ein Fällbad bestehend aus einer 100 mM CaCl₂ Lösung oder 20 mM BaCl₂ Lösung vertropft.

Anschließend wurden die Kapseln (Beads) 5mal mit 10ml Ringer Lösung gewaschen. Danach wurden die erhaltenen Kapseln (Beads) mit einem mittleren Durchmesser von 450 µm in Ringer Lösung aufgenommen und je 500 µl in 1ml Fertigspritzen abgefüllt.

Die steril hergestellten Fertigspritzen wurden bei 5° C plus/minus 3° C gelagert.

Für die Injektion wurde eine 21 G Nadel verwendet.

### 3. Subcutane Testung an der Ratte

### a) Subcutane Implantation

Ratten, die gemäß internationalen Richtlinien für die Testung der subcutanen Verträglichkeit empfohlen werden, wurden entsprechend ausgewählt, vorbereitet und unter Anästhesie subcutan mit den jeweiligen Prüfsubstanzen und Referenzsubstanzen (flüssige Alginatlösung bzw. aufgereinigte Beads, siehe Herstellung unter Beispiel 2) unterspritzt.

Dazu wurden jedem Tier an den 4 Teststellen jeweils die verschiedenen Prüfsubstanzen und Referenzsubstanzen (flüssige Alginatlösung bzw. aufgereinigte Beads, siehe Herstellung unter Beispiel 2) subkutan 0,40 plus/minus 0,05 ml Produkt so injiziert, dass durch Herausziehen der Nadel das Produkt über eine Strecke von 2cm verteilt wird. Bei und nach dem Herausziehen wurde eine entsprechende längliche Schwellung sichtbar. Die Injektionsstellen wurden anschließend durch ein Tattoo markiert.

Der Zustand der Tiere und der Injektionsstellen wurde anfänglich alle zwei Tage, später zweimal wöchentlich untersucht.

### b) Explantation direkt nach Implantation

Die Applikation der jeweiligen Prüfsubstanzen und Referenzsubstanzen resultierte in einer gut tastbaren Schwellung.

In einem zusätzlich zu dieser Studie durchgeführten Experiment wurde das nach Injektion der erfindungsgemäß hergestellten hochreinen, unvernetzten und hochmolekularen Alginatlösung (Prüfsubstanz 1) 60 Minuten nach Injektion wieder explantiert. Zu diesem Zeitpunkt war die Prüfsubstanz bereits durch endogenes Calcium vernetzt und als Gelkissen explantierbar (siehe Abbildung 1).

Wie in Abbildung 1 zu sehen ist, führt die Verabreichung der Prüfsubstanz 1 (unvernetzte Alginatlösung) 60 Minuten nach Applikation zu einem vollständig vernetzten Implantat. Die subkutan injizierte Alginatlösung zeigt sich dabei auch nach der Freipräparation der Haut makroskopisch als Schwellung (siehe Fig. 1 A). Ferner ist die applizierte Prüfsubstanz 1 gut tastbar und formstabil (siehe Fig. 1 B). Der Versuch belegt zudem, dass die ehemals flüssige Alginatlösung bereits 60 Minuten nach Applikation durch endogenes Calcium vernetzt und als Gelkissen explantierbar ist (siehe Fig. 1 C).

### 4. Explantation und Pathologie der Implantationsorte

Je 6 Ratten wurden nach 7 Tagen, 3 und 6 Monaten euthanasiert. Alle Injektionsstellen wurden geöffnet und auf die Anwesenheit und Beschaffenheit des Implantats geprüft.

Die Implantate wurden visuell und auf Ca²⁺ Gehalt untersucht. Alle Implantate waren nach allen drei Zeitpunkten vollständig und klar abgegrenzt sichtbar und isolierbar. Die Messung des Ca²⁺-Gehalts der explantierten Beads war nur annähernd möglich, da der Serumgehalt der explantierten Beads unterschiedlich sein kann.

Die aus Alginatlösung entstandenen monolitischen Implantate (Gesamtvolumen ungefähr 0,4ml) zeigen bereits bei der 1. Explantation nach 7 Tagen gleich hohe Ca²⁺ Werte wie die extern Ca²⁺-vernetzten Beads und bleiben in ihrem Ca²⁺ Gehalt über die Beobachtungszeit von 6 Monaten konstant (250-330 mg Ca²⁺/g Implantat)

Die histopathologische Untersuchung zeigte ferner, dass das monolithische Implantat mindestens so gewebeverträglich ist wie die Ca²⁺-vernetzten Beads.

### • Ergebnis nach 1 Woche:

Die subkutane Injektion der Alginatlösung in die Ratte zeigte nach einer Woche eine leicht entzündliche Reaktion (leichte Infiltration mit Makrophagen, Lymphozyten und Plasmazellen und zu einem geringeren Anteil Riesenzellen) im Vergleich zur Implantation von Beads, jedoch wurde eine geringere Infiltration mit Lymphozyten im Vergleich zur Bead-Implantation beobachtet. Polymorphonukleäre Zellen und Nekrose wurden nicht beobachtet. Es wurde lediglich eine leichte Fibrose und Fibroplasie nach Injektion der Alginatlösung in die Ratte festgestellt. Innerhalb des Alginat-Depots können spiralartige Fasern, offensichtlich Kollagenfasern, detektiert werden.

Es konnte eine leichte Neovaskularisation, ein kaum feststellbarer Abbau sowie ein Fibrin-Einschluß nach der Injektion der Alginatlösung beobachtet werden. Es erfolgte keine Einkapselung des Alginats und keine Degeneration des Gewebes aufgrund der Injektion der Alginatlösung.

### • Ergebnis nach 3 Monaten:

Die entzündliche Reaktion, die durch die injizierte Alginatlösung hervorgerufen wurde, zeigte nach drei Monaten eine ähnliche Natur wie nach einer Woche, mit der Ausnahme, dass drei Monate nach Injektion keine Riesenzellen auftraten.

Das Ausmaß der Fibroplasie wie auch der Fibrose rund um das Alginat-Depot wurde reduziert und es wurde beobachtet, dass in geringem Maße vorhandenes fibrotisches Gewebe in zwei von drei Fällen eine dünne Kapsel mit Fibrin bildet. Ähnlich zum früheren Zeitpunkt wurde eine leichte Neovaskularisation festgestellt.

### • Ergebnis nach 6 Monaten:

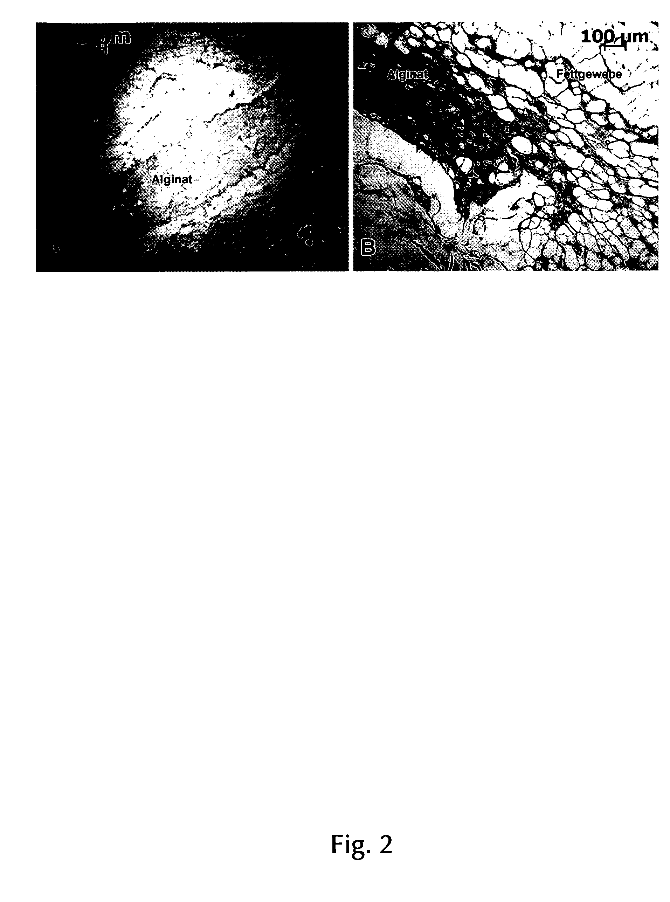

Sechs Monate nach Injektion war das Alginat noch immer als solides bis netzwerkartiges Depot mit eingebetteten Kollagenfasern vorhanden (siehe Figur 2) und zeigte eine gute Bioverträglichkeit. Die Entzündung als auch die fibrotische Reaktion, die durch das Alginatimplantat verursacht wurden, war sechs Monate nach der Injektion der Alginatlösung wesentlich besser im Vergleich zur Biokompatibilität nach drei Monaten. Keine der Injektionsstellen zeigte eine fibrotische Reaktion und die entzündliche Antwort bestand lediglich aus einigen Plasmazellen und Makrophagen. Es wurden keine weiteren entzündlichen Antworten induziert (siehe Figur 2). Figur 2 zeigt dabei H&E Färbungen der Stellen der Injektion von Alginaten 6 Monate nach der subkutanen Injektion in die Ratte. In Figur 2A ist eine Übersicht über ein Alginat Depot dargestellt, Figur 2B zeigt ein Detail eines netzartigen Auswuchses des Alginat Depots innerhalb des Fettgewebes mit eingebetteten Kollagenfasern. Damit zeigt sich im Gesamten eine offensichtlich sehr gute Bioverträglichkeit des ausschließlich durch endogenes Ca²⁺ *in situ* vernetzten Alginatimplantats.

### 5. Subcutane Testung am Kaninchen

200 µl einer 1%-igen hochreinen Alginatlösung, die wie oben beschrieben erfindungsgemäß hergestellt wurde, wurden mittels einer 1ml Spritze an links und rechts je 6 Injektionsstellen am Rücken der rasierten Kaninchen mit einer 30G-Nadel injiziert.

Ziel der Testung war die Beurteilung der Stabilität und lokalen Verträglichkeit von zwei Alginatpräparationen und einem handelsüblichen Vergleichsprodukt eines chemisch vernetzten partikularen Polysaccharins. Das hier verwendete Testdesign wird gemäß internationalen Richtlinien für Tests dieser Art empfohlen.

### 6. Ergebnisse der Versuche aus Beispiel 5

Die nach Euthanasie nach 4 und 12 Wochen entnommenen Injektionsstellen wurden histopathologisch untersucht. An allen Injektionsstellen waren Alginathydrogelkörper in das subkutane Gewebe der Versuchstiere eingebettet. Das Material zeigte eine hohe Gewebeintegration und eine sehr gute Verträglichkeit der Testimplantate.

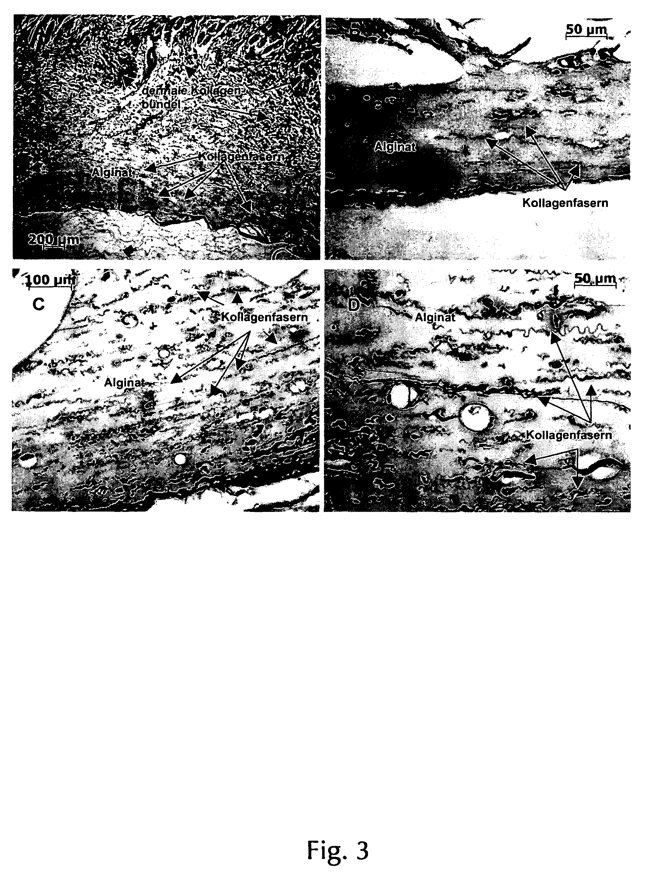

Die entsprechenden van Gieson-Färbungen der Entnahmen nach 4 Wochen sind in Figur 3 A-D dokumentiert. Figur 3 zeigt dabei Kollagenfärbungen (van Gieson Färbung) der Injektionssstellen mit den Testimplantaten 1 (A, B) und 2 (C, D) vier Wochen nach der Injektion in das Kaninchen. Insbesondere zeigt Figur 3A Kollagenfasern innerhalb des i.d. injizierten Testimplantats 1; die Kollagenfasern sind gleichmäßig rubinrot gefärbt wie die ursprünglichen dermalen Kollagenbündel. Figur 3B zeigt eine detaillierte Mikroaufnahme des s.c. implantierten Testimplantats 1, das mit Kollagenfasern durchsetzt ist. Figur 3C zeigt ein s.c. implantiertes Testimplantat 2, das mit einer Vielzahl von Kollagenfasern durchsetzt ist, und Figur 3D beschreibt eine detaillierte Mikroaufnahme des s.c. implantierten Testimplantats 2, das mit Kollagenfasern verschiedener Länge durchsetzt ist.

Es konnte damit nicht nur gezeigt werden, dass das monolithische Alginat-Implantat, das gemäß der vorliegenden Erfindung hergestellt wurde, gegenüber der Umgebung sehr verträglich ist, sondern auch das Einwachsen von Kollagenfibrillen begünstigt.

Die Ergebnisse nach 12 Wochen entsprechen analog den 4 Wochenergebnissen; d.h. die monolithischen Alginat-Implantate sind völlig intakt wiederzufinden. In einigen Fällen ist das Implantat von einer dünnen Schicht Fibroblasten umgeben.

Im Ergebnis zeigen die gemäß der vorliegenden Erfindung injizierten Alginatlösungen eine rasche endogene Ca²⁺-Vernetzung und die gebildeten monolithischen Alginat-Implantate sind 4 und 12 Wochen nach Injektion vollständig intakt wiederzufinden. Die Verträglichkeit der monolithischen Alginat-Implantate ist in allen Fällen so gut wie die des Handelsproduktes (Referenzprodukt, Beads); darüber hinaus waren eingewachsene Kollagenfibrillen klar darstellbar.

### 7. Auspressdruck (kosmetischer) Filler

Im folgenden Versuch wurde der auf die Spritze ausgeübte Druck in Abhängigkeit vom Produkt gemessen. Dabei wurden verschiedene Chargen von Alginatsol, wie unter Experiment/Beispiel 1 beschrieben, untersucht. Der notwendige Druck lag in allen 6 Proben bei 7-8 Newton, gemessen mit einer 30 G-Nadel (siehe Abbildung 4), d.h. in einem leicht zu handhabbaren Bereich.

Die Auspressdrucke zweier verschiedener, kommerziell erhältlicher Hyaluronsäure-Präparate lag dagegen bei einer 27 G-Nadel bzw. 30 G Nadel in beiden Fällen über 25 Newton.

### 8. Mechanische Stabilität von monolithischen Alginat-Implantaten

Der Versuchsaufbau ist analog zu Beispiel 3. Ziel des vorliegenden Versuches war es, nachzuweisen, wie sich die Stabilität monolithischer Alginat-Implantate in Abhängigkeit von Alginat und im Vergleich zu Alginatsbeads aus Beispiel 2 darstellt.

Abbildung 5 zeigt die über 90 Tage verfolgte Implantatgröße ermittelt durch Abtastung mit einer Schieblehre. Prüfsubstanz 1 ist hier ein hochmolekulares Alginat (MW > 500.000 g/mol), wie in dieser Erfindung beansprucht. Prüfsubstanz 2 ist nieder molekulares Alginat mit einem mittleren MG von unter 120.000 Dalton. Die Referenzsubstanzen 1 und 2 sind Alginatbeads, wie in Beispiel 2 beschrieben, allerdings mit einem mittleren Durchmesser von 500 µm.

Fig. 5 zeigt in diesem Zusammenhang die Darstellung der Mittelwerte der ertasteten Maximal- und Minimaldurchmesser der verschiedenen Test-Implantate (Prüfsubstanzen und Referenzsubstanzen) im Versuchszeitraum. Das Implantat wurde am narkotisierten Tier ertastet und die Implantatgröße mit Hilfe einer Schieblehre vermessen.
Prüfsubstanz 1 (unvernetzte Alginatlösung);
Prüfsubstanz 2 (niedermolekulare unvernetzte Alginatlösung),
Referenzsubstanz 1 (CellBeads^{®} 500),
Referenzsubstanz 2 (CellBeads^{®} 500, autoklaviert).

Die Daten resultieren aus dem Vermessen folgender Implantatstellen: Tag 1 n=16, Tag 2-7 je n=12, Tag 14 -28 je n=8, verbleibende Tage je n=4.

Zusammenfassend zeigt dieses Beispiel deutlich den Einfluss der *in vivo*-Haltbarkeit des *in vivo*-vernetzten monolithischen Alginat-Implantats.

## Patentansprüche

1. Verwendung eines Alginats zur Herstellung einer unvernetzten, hochreinen und hochmolekularen Alginatlösung (Sol) als Füllmaterial in der Medizin oder Kosmetik zum Zweck der Volumenauffüllung, wobei das Alginat in der unvernetzten, hochreinen und hochmolekularen Alginatlösung (Sol) in einer Konzentration von 0,5-2,5% (w/v) Alginat Feststoffanteil vorliegt und die *in vivo* injizierte, unvernetzte, hochreine und hochmolekulare Alginatlösung *in situ* zur spontanen Ca²⁺ Vernetzung ohne exogene Zugabe eines Vernetzers führt.

2. Verwendung nach Anspruch 1, **dadurch gekennzeichnet, dass** das Alginat ausgewählt wird aus hochreinen und hochmolekularen Alginaten mit einer mittleren Molmasse von über 200.000 Dalton, bevorzugt aus hochreinen und hochmolekularen Alginaten mit einer mittleren Molmasse von 200.000 bis 5.000.000 Dalton, stärker bevorzugt aus hochreinen und hochmolekularen Alginaten mit einer mittleren Molmasse von 200.000 bis 1.000.000 Dalton, und am stärksten bevorzugt aus hochreinen und hochmolekularen Alginaten mit einer mittleren Molmasse von 200.000 bis 500.000 Dalton

3. Verwendung nach Anspruch 1 oder 2, **dadurch gekennzeichnet, dass** als Alginat ein hochreines und hochmolekulares Kalium- oder Natriumalginat verwendet wird.

4. Verwendung nach Anspruch 1 bis 3, **dadurch gekennzeichnet, dass** sich nach Injektion *in situ* monolithische Alginatimplantatkörper am Injektionsort bilden.

5. Verwendung nach einem der Ansprüche 1 bis 4, **dadurch gekennzeichnet, dass** die unvernetzte, hochreine und hochmolekulare Alginatlösung (Sol) in einer Konzentration von 0,6-1,0% (w/v) Alginat Feststoffanteil, bezogen auf das Gesamtgewicht, vorliegt.

6. Verwendung nach einem der Ansprüche 1 bis 5, **dadurch gekennzeichnet, dass** das Alginat der unvernetzten, hochreinen und hochmolekularen Alginatlösung (Sol) in einer physiologischen Injektionslösung suspendiert ist.

7. Verwendung nach einem der Ansprüche 1 bis 6, **dadurch gekennzeichnet, dass** die unvernetzte, hochreine und hochmolekulare Alginatlösung (Sol) Wirkstoffe enthält, die ausgewählt werden aus der Gruppe, bestehend aus pharmazeutisch wirksamen Verbindungen, Nährstoffen, Markersubstanzen, vitalen Zellen, und wasserlöslichen Hilfsmitteln oder Stabilisierungsmitteln.

8. Verwendung nach Anspruch 7, **dadurch gekennzeichnet, dass** die pharmazeutisch wirksamen Verbindungen ausgewählt werden aus den Substanzklassen der Vitamine, Adhäsionsproteine, der entzündungshemmenden Substanzen, der Antibiotika, Analgetika, Wachstumsfaktoren, Hormone, besonders bevorzugt ausgewählt aus Protein- und/oder Peptid-basierten Wirkstoffen, einschließlich humanes Wachstumshormon (human growth hormone), Rinderwachstumshormon (bovine growth hormone), Wachstumshormon aus Schwein (porcine growth hormone), Growth hormone releasing hormone/peptide, Granulozyten-Kolonien stimulierender Faktor (granulocyte-colony stimulating factor), Granulozyten-Makrophagen-Kolonien stimulierender Faktor (granulocyte macrophage-colony stimulating factor), Makrophagen stimulierender Faktor (macrophage-colony stimulating factor), Erythropoietin, Bone morphogenic protein, Interferon oder Derivaten davon, Insulin oder Derivaten davon, Atriopeptin-III, monoklonale Antikörper, Tumornekrosefaktor, Macrophagen aktivierender Faktor (macrophage activating factor), Interleukin, Tumor degenerierender Faktor (tumor degenerating factor), Insulin-like growth factor, Epidermaler Wachstumsfaktor (epidermal growth factor), Gewebe-Plasminogen-Aktivator (tissue plasminogen activator), Faktor MV, Faktor IMV, und Urokinase.

9. Verwendung nach Anspruch 7, **dadurch gekennzeichnet, dass** die wasserlöslichen Hilfsmittel oder Stabilisierungsmittel ausgewählt werden aus der Gruppe, bestehend aus Proteinen, einschließlich Albumin oder Gelatin; Aminosäuren, einschließlich Glycin, Alanin, Glutaminsäure, Arginin, Lysin oder ein Salz davon; Kohlenhydraten, einschließlich Glucose, Lactose, Xylose, Galaktose, Fruktose, Maltose, Saccharose, Dextran, Mannitol, Sorbitol, Trehalose und Chondroitinsulfat; anorganischen Salzen, einschließlich Phosphat; Benetzungsmitteln, einschließlich TWEEN^{®} (ICI), Polyethylenglykol, oder einer Mischung davon.

10. Verwendung nach einem der Ansprüche 1 bis 9, **dadurch gekennzeichnet, dass** in der unvernetzten, hochreinen und hochmolekularen Alginatlösung (Sol) mindestens eine weitere Substanz mit Fülleigenschaften enthalten ist.

11. Verwendung nach Anspruch 10, **dadurch gekennzeichnet, dass** die mindestens eine weitere Substanz mit Fülleigenschaften in Gewichtsanteilen von 5-50% vom Filler-Gesamtgewicht (w/w) in der unvernetzten, hochreinen und hochmolekularen Alginatlösung (Sol) enthalten ist.

12. Verwendung nach Anspruch 10 oder 11, **dadurch gekennzeichnet, dass** die mindestens eine weitere Substanz mit Fülleigenschaften ausgewählt wird aus der Gruppe, bestehend aus Hyaluronsäure oder einem Salz davon, Kollagen und Polyacrylamid in löslicher Form.

13. Verwendung nach Anspruch 12, **dadurch gekennzeichnet, dass** die Hyaluronsäure eine unvernetzte, hochgereinigte Hyaluronsäure oder ein Salz davon mit einem Molekulargewicht im Bereich von 10.000 Da bis 10.000.000 Da, bevorzugt einem Molekulargewicht im Bereich von 25.000 Da bis 5.000.000 Da und am stärksten bevorzugt einem Molekulargewicht im Bereich von 50.000 Da bis 3.000.000 Da ist.

14. Verwendung nach einem der Ansprüche 12 oder 13, **dadurch gekennzeichnet, dass** das Salz der unvernetzten, hochgereinigten Hyaluronsäure ausgewählt wird aus Natriumhyaluronat, Kaliumhyaluronat oder Ammoniumhyaluronat.

15. Verwendung nach einem der Ansprüche 10 oder 11, **dadurch gekennzeichnet, dass** die mindestens eine weitere Substanz mit Fülleigenschaften eine feste, in der unvernetzten, hochreinen und hochmolekularen Alginatlösung (Sol) unlösliche Substanz mit einer Partikelgröße zwischen 10 und 150 µm ist.

16. Verwendung nach Anspruch 15, **dadurch gekennzeichnet, dass** die unlösliche Substanz PMMA-Mikropartikel, Polymilchsäurepartikel, HEMA-Partikel, Calciumhydroxylapatitpartikel in möglichst runder Partikelform mit einem Durchmesser von 10-80 µm sind.

17. Verwendung nach Anspruch 15 oder 16, **dadurch gekennzeichnet, dass** unlösliche Substanz faserförmig ausgebildet ist.

18. Verwendung nach Anspruch 15 bis 17, **dadurch gekennzeichnet, dass** als unlösliche Substanz Stapelfasern mit einem Durchmesser von 5-40 µm und einer Länge von 20-100 µm verwendet werden.

19. Verwendung nach Anspruch 17 oder 18, **dadurch gekennzeichnet, dass** die Fasern aus gewebeverträglichen, im Körper abbaubaren Polymeren bestehen.

20. Verwendung nach einem der Ansprüche 17 bis 19, **dadurch gekennzeichnet, dass** die Fasern aus Collagen, Polymilchsäure und deren Copolymere (mit Glycin), kovalent vernetzter Hyaluronsäure, Alginsäure, oder Acryl- und Methacrylsäureesterpolymeren bestehen.

21. Verwendung nach einem der Ansprüche 11 bis 12, **dadurch gekennzeichnet, dass** die mindestens eine weitere Substanz mit Fülleigenschaften ausgewählt wird aus autologen Bestandteilen, umfassend Zellen, Plasmaproteine oder Liposuccionmaterial.

22. Verwendung gemäß einem der Ansprüche 1 bis 21, **dadurch gekennzeichnet, dass** das gebildete monolithische Alginatimplantat durch Injektion einer EDTA- oder Citratlösung oder einer Lösung aus Komplexbildnern wieder aufgelöst werden kann oder wird.

23. Verwendung gemäß einem der Ansprüche 1 bis 22 zur Behandlung von Hautfalten.

24. Verwendung gemäß einem der Ansprüche 1 bis 22 zur Unterstützung von Sphinktermuskulaturen.

25. Verwendung gemäß einem der Ansprüche 1 bis 22 zur Behandlung der gastroösophagealen Refluxkrankheit.

26. Verwendung gemäß einem der Ansprüche 1 bis 22 zur Behandlung von Harninkontinenz.

27. Verwendung gemäß einem der Ansprüche 1 bis 22 zur Behandlung der vesiko-ureteralen Refluxkrankheit.

28. Verwendung gemäß einem der Ansprüche 1 bis 22 in der Wiederherstellungschirurgie.

29. Verwendung gemäß einem der Ansprüche 1 bis 22 zur Behandlung von Tumoren.

30. Verwendung gemäß einem der Ansprüche 1 bis 22 zur kosmetischen Verwendung.
